# EUROPEAN PATENT APPLICATION

(11) **EP 4 206 198 A1**
(43) Date of publication of application: **05.07.2023**
(21) Application number: 21861904.7
(22) Date of filing: 30.07.2021
(51) Int. Cl.: C07D 405/04, C07D 495/04, C07D 491/048, C07D 409/04, H01L 51/00, H01L 51/50

(54) **HETEROCYCLIC COMPOUND, ORGANIC LIGHT-EMITTING DEVICE COMPRISING SAME, AND COMPOSITION FOR ORGANIC MATERIAL LAYER OF ORGANIC LIGHT-EMITTING DEVICE**

(30) Priority: 25.08.2020 KR 20200106968
(71) Applicant: LT Materials Co., Ltd., Yongin-si, Gyeonggi-do 17118 (KR)
(72) Inventor: KWON, Su-Jin, Yongin-si, Gyeonggi-do 17118 (KR); CHOI, Eui-Jeong, Yongin-City, Gyeonggi-do 17118 (KR); KIM, Ji-Young, Yongin-si, Gyeonggi-do 17118 (KR); MO, Jun-Tae, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/009979
(87) International publication number: WO 2022/045606

(57) **Abstract**

The present application provides a heterocyclic compound, an organic light emitting device comprising the heterocyclic compound in an organic material layer, and a composition for an organic material layer.

## Description

### [Technical Field]

This application claims priority to and the benefits of Korean Patent Application No. 10-2020-0106968, filed with the Korean Intellectual Property Office on August 25, 2020, the entire contents of which are incorporated herein by reference.

The present specification relates to a heterocyclic compound, an organic light emitting device comprising the same, and a composition for an organic material layer of an organic light emitting device.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### Prior Art Documents

### Patent Documents

US Patent No. 4,356,429

### [Disclosure]

### [Technical Problem]

The present disclosure is directed to providing a heterocyclic compound, an organic light emitting device comprising the same, and a composition for an organic material layer of an organic light emitting device.

### [Technical Solution]

One embodiment of the present application provides a heterocyclic compound represented by the following Chemical Formula 1.

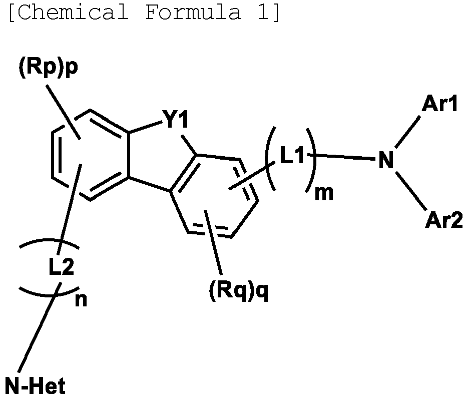

In Chemical Formula 1,
Y1 is O; or S,
L1 and L2 are the same as or different from each other, and each independently a direct bond; a substituted or unsubstituted arylene group having 6 to 60 carbon atoms; or a substituted or unsubstituted heteroarylene group having 2 to 60 carbon atoms, m and n are each an integer of 0 to 3, and when m and n are each 2 or greater, substituents in the parentheses are the same as or different from each other,
N-Het is a monocyclic or polycyclic heterocyclic group substituted or unsubstituted and comprising one or more Ns,
Ar1 and Ar2 are the same as or different from each other, and each independently a substituted or unsubstituted aryl group having 6 to 60 carbon atoms; or a substituted or unsubstituted heteroaryl group having 2 to 60 carbon atoms, and
Rp and Rq are the same as or different from each other, and each independently hydrogen; deuterium; a halogen group; or a substituted or unsubstituted alkyl group having 1 to 60 carbon atoms, p and q are each an integer of 0 to 3, and when p and q are each 2 or greater, substituents in the parentheses are the same as or different from each other.

In addition, another embodiment of the present application provides an organic light emitting device comprising a first electrode, a second electrode, and one or more organic material layers provided between the first electrode and the second electrode, wherein one or more layers of the organic material layers comprise the heterocyclic compound represented by Chemical Formula 1.

### [Advantageous Effects]

A heterocyclic compound according to one embodiment of the present application can be used as a material of an organic material layer of an organic light emitting device. The heterocyclic compound can be used as a material of a hole injection layer, a hole transfer layer, a light emitting layer, an electron transfer layer, an electron injection layer, a charge generation layer and the like in an organic light emitting device. Particularly, the heterocyclic compound represented by Chemical Formula 1 can be used as a material of a light emitting layer of an organic light emitting device. In addition, using the heterocyclic compound represented by Chemical Formula 1 in an organic light emitting device is capable of lowering a driving voltage of the device, enhancing light efficiency, and enhancing lifetime properties of the device by thermal stability of the compound.

### [Description of Drawings]

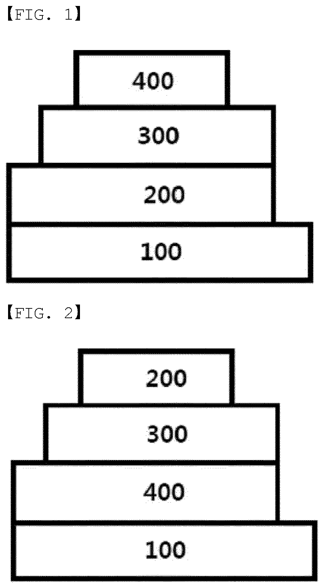

FIG. 1 to FIG. 4 are diagrams each schematically illustrating a lamination structure of an organic light emitting device according to one embodiment of the present application.

### [Mode for Disclosure]

Hereinafter, the present application will be described in detail.

One embodiment of the present application provides a heterocyclic compound represented by the following Chemical Formula 1.

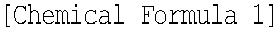

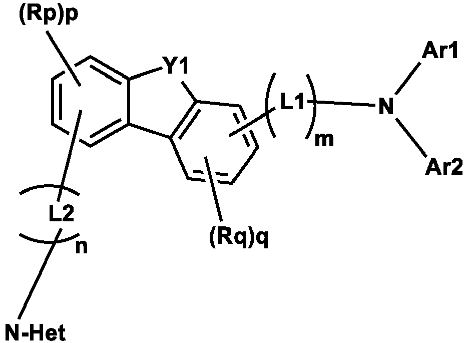

In Chemical Formula 1,
Y1 is O; or S,
L1 and L2 are the same as or different from each other, and each independently a direct bond; a substituted or unsubstituted arylene group having 6 to 60 carbon atoms; or a substituted or unsubstituted heteroarylene group having 2 to 60 carbon atoms, m and n are each an integer of 0 to 3, and when m and n are each 2 or greater, substituents in the parentheses are the same as or different from each other,
N-Het is a monocyclic or polycyclic heterocyclic group substituted or unsubstituted and comprising one or more Ns,
Ar1 and Ar2 are the same as or different from each other, and each independently a substituted or unsubstituted aryl group having 6 to 60 carbon atoms; or a substituted or unsubstituted heteroaryl group having 2 to 60 carbon atoms, and
Rp and Rq are the same as or different from each other, and each independently hydrogen; deuterium; a halogen group; or a substituted or unsubstituted alkyl group having 1 to 60 carbon atoms, p and q are each an integer of 0 to 3, and when p and q are each 2 or greater, substituents in the parentheses are the same as or different from each other.

By having an amine group and a monocyclic or polycyclic heterocyclic group comprising one or more Ns as substituents in the dibenzofuran or dibenzothiophene structure, Chemical Formula 1 is more electron abundant, and, by improving a current flow, an effect of lowering a driving voltage is obtained when using the compound represented by Chemical Formula 1 in a device. In addition, by having an amine group having hole properties as a substituent, Chemical Formula 1 has an excellent hole transfer ability, and an effect of lowering a driving voltage is obtained when using the compound represented by Chemical Formula 1 in a device.

In the present specification, the term "substitution" means, instead of a hydrogen atom bonding to a carbon atom of a compound, another substituent bonding thereto, and the position of substitution is not limited as long as it is a position at which the hydrogen atom bonds, that is, a position at which a substituent is capable of bonding, and when two or more substituents substitute, the two or more substituents may be the same as or different from each other.

In the present specification, the "substituted or unsubstituted" means being substituted with one or more substituents selected from the group consisting of deuterium; a cyano group; a halogen group; linear or branched alkyl having 1 to 60 carbon atoms; linear or branched alkenyl having 2 to 60 carbon atoms; linear or branched alkynyl having 2 to 60 carbon atoms; monocyclic or polycyclic cycloalkyl having 3 to 60 carbon atoms; monocyclic or polycyclic heterocycloalkyl having 2 to 60 carbon atoms; monocyclic or polycyclic aryl having 6 to 60 carbon atoms; monocyclic or polycyclic heteroaryl having 2 to 60 carbon atoms; -SiRR'R"; -P(=O)RR'; alkylamine having 1 to 20 carbon atoms; monocyclic or polycyclic arylamine having 6 to 60 carbon atoms; and monocyclic or polycyclic heteroarylamine having 2 to 60 carbon atoms, or being unsubstituted, or being substituted with a substituent linking two or more substituents selected from among the substituents illustrated above, or being unsubstituted, and R, R' and R" are the same as or different from each other and each independently substituted or unsubstituted alkyl having 1 to 60 carbon atoms; substituted or unsubstituted aryl having 6 to 60 carbon atoms; or substituted or unsubstituted heteroaryl having 2 to 60 carbon atoms.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one Example, having a deuterium content of 20% in a phenyl group represented by 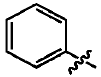 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

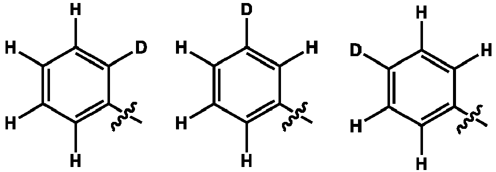

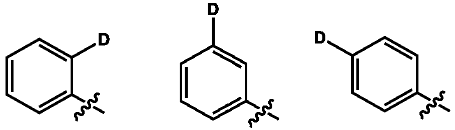

In addition, in one embodiment of the present application, "a phenyl group having a deuterium content of 0%" may mean a phenyl group that does not comprise a deuterium atom, that is, a phenyl group that has 5 hydrogen atoms.

In the present specification, the halogen may be fluorine, chlorine, bromine or iodine.

In the present specification, the alkyl group includes linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide group may include a diphenylphosphine oxide group, a dinaphthylphosphine oxide group and the like, but are not limited thereto.

In the present specification, the silyl group is a substituent including Si, having the Si atom directly linked as a radical, and is represented by -SiR104R105R106. R104 to R106 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the fluorenyl group may be substituted, and adjacent substituents may bond to each other to form a ring.

In the present specification, the spiro group is a group including a spiro structure, and may have 15 to 60 carbon atoms. For example, the spiro group may include a structure in which a 2,3-dihydro-1H-indene group or a cyclohexane group spiro bonds to a fluorenyl group. Specifically, the spiro group may include any one of groups of the following structural formulae.

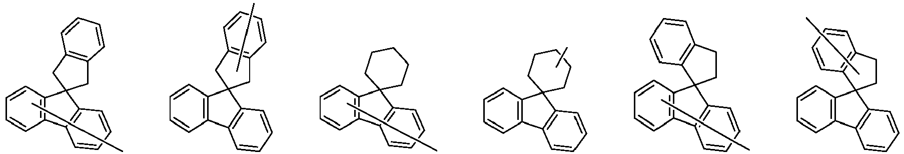

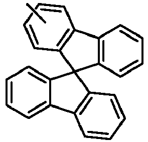

In the present specification, the heteroaryl group includes S, O, Se, N or Si as a heteroatom, includes monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, 5,10-dihydrobenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto. In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except that these are each a divalent group. In addition, the heteroarylene group means the heteroaryl group having two bonding sites, that is, a divalent group. The descriptions on the heteroaryl group provided above may be applied thereto except that these are each a divalent group.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

The heterocyclic compound according to one embodiment of the present application is represented by Chemical Formula 1. More specifically, by having a core structure and structural properties of the substituents as above, the heterocyclic compound represented by Chemical Formula 1 may be used as a material of an organic material layer of an organic light emitting device.

In one embodiment of the present application, Y1 of Chemical Formula 1 is O; or S.

In another embodiment, Y1 is O.

In another embodiment, Y1 is S.

In one embodiment of the present application, L1 and L2 of Chemical Formula 1 are the same as or different from each other, and may be each independently a direct bond; a substituted or unsubstituted arylene group having 6 to 60 carbon atoms; or a substituted or unsubstituted heteroarylene group having 2 to 60 carbon atoms.

In one embodiment of the present application, L1 and L2 are the same as or different from each other, and may be each independently a direct bond; a substituted or unsubstituted arylene group having 6 to 40 carbon atoms; or a substituted or unsubstituted heteroarylene group having 2 to 40 carbon atoms.

In one embodiment of the present application, L1 and L2 are the same as or different from each other, and may be each independently a direct bond; a substituted or unsubstituted arylene group having 6 to 20 carbon atoms; or a substituted or unsubstituted heteroarylene group having 2 to 20 carbon atoms.

In one embodiment of the present application, L1 may be a direct bond; a substituted or unsubstituted arylene group having 6 to 60 carbon atoms; or a substituted or unsubstituted heteroarylene group having 2 to 60 carbon atoms.

In another embodiment, L1 is a direct bond.

In another embodiment, L1 is an arylene group having 6 to 60 carbon atoms; or a substituted or unsubstituted heteroarylene group having 2 to 60 carbon atoms.

In another embodiment, L1 is a phenylene group.

In one embodiment of the present application, L2 may be a direct bond; a substituted or unsubstituted arylene group having 6 to 60 carbon atoms; or a substituted or unsubstituted heteroarylene group having 2 to 60 carbon atoms.

In another embodiment, L2 is a direct bond.

In another embodiment, L2 is an arylene group having 6 to 60 carbon atoms; or a substituted or unsubstituted heteroarylene group having 2 to 60 carbon atoms.

In another embodiment, L2 is a phenylene group.

In one embodiment of the present application, m and n of Chemical Formula 1 are each an integer of 0 to 3, and when m and n are each 2 or greater, substituents in the parentheses may be the same as or different from each other.

In another embodiment, m is 0.

In another embodiment, m is 1.

In another embodiment, m is 2.

In another embodiment, m is 3.

In one embodiment of the present application, when m is 2 or greater, substituents in the parentheses may be the same as or different from each other.

In another embodiment, n is 0.

In another embodiment, n is 1.

In another embodiment, n is 2.

In another embodiment, n is 3.

In one embodiment of the present application, when n is 2 or greater, substituents in the parentheses may be the same as or different from each other.

In one embodiment of the present application, N-Het of Chemical Formula 1 may be a monocyclic or polycyclic heterocyclic group substituted or unsubstituted and comprising one or more Ns.

In one embodiment of the present application, N-Het may be a group represented by the following Chemical Formula 3.

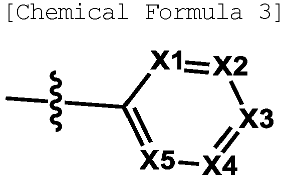

In Chemical Formula 3,
X1 is CR1 or N, X2 is CR2 or N, X3 is CR3 or N, X4 is CR4 or N, X5 is CR5 or N, and at least one of X1 to X5 is N, and
R1 to R5 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; -P(=O)R10R12; and NR13R14, or two or more groups adjacent to each other bond to each other to form a substituted or unsubstituted aliphatic or aromatic hydrocarbon ring or heteroring, R10 to R14 are the same as or different from each other and each independently hydrogen; deuterium; a halogen group; a substituted or unsubstituted alkyl group having 1 to 60 carbon atoms; a substituted or unsubstituted aryl group having 6 to 60 carbon atoms; or a substituted or unsubstituted heteroaryl group having 2 to 60 carbon atoms, and 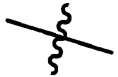 is a site linked to Chemical Formula 1.

In one embodiment of the present application, Chemical Formula 3 may be represented by one of the following Chemical Formulae 3-1 to 3-4. Herein, 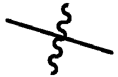 is a site linked to Chemical Formula 1.

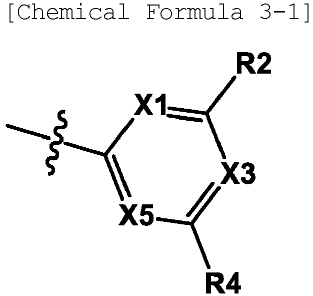

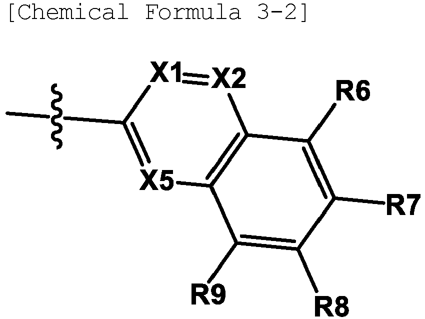

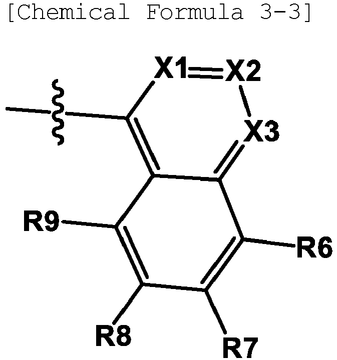

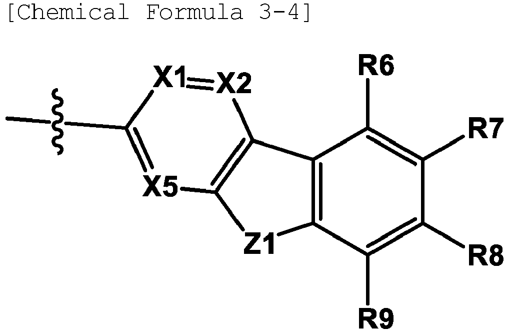

In Chemical Formula 3-1, at least one of X1, X3 and X5 is N, and the rest have the same definitions as in Chemical Formula 3,
in Chemical Formula 3-2, at least one of X1, X2 and X5 is N, and the rest have the same definitions as in Chemical Formula 3,
in Chemical Formula 3-3, at least one of X1 to X3 is N, and the rest have the same definitions as in Chemical Formula Chemical Formula 3,
in Chemical Formula 3-4, at least one of X1, X2 and X5 is N, and the rest have the same definitions as in Chemical Formula 3,
Z1 is O; or S, and
R2, R4 and R6 to R9 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; -P(=O)R10R12; and NR13R14, or two or more groups adjacent to each other bond to each other to form a substituted or unsubstituted aliphatic or aromatic hydrocarbon ring or heteroring, and R10 to R14 are the same as or different from each other and each independently hydrogen; deuterium; a halogen group; a substituted or unsubstituted alkyl group having 1 to 60 carbon atoms; a substituted or unsubstituted aryl group having 6 to 60 carbon atoms; or a substituted or unsubstituted heteroaryl group having 2 to 60 carbon atoms.

In one embodiment of the present application, Chemical Formula 3 may be represented by any one of the following Group A.

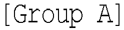

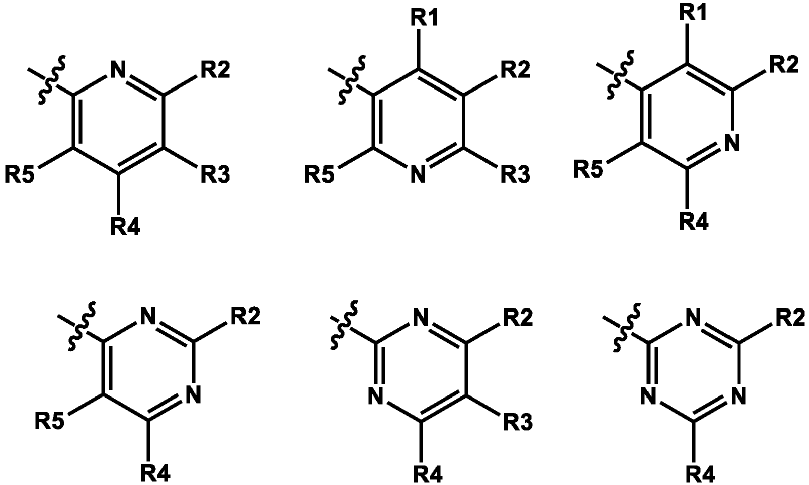

In Group A, R1 to R5 and 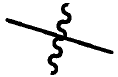 have the same definitions as in Chemical Formula 3.

In one embodiment of the present application, Ar1 and Ar2 of Chemical Formula 1 are the same as or different from each other, and may be each independently a substituted or unsubstituted aryl group having 6 to 60 carbon atoms; or a substituted or unsubstituted heteroaryl group having 2 to 60 carbon atoms.

In one embodiment of the present application, Ar1 and Ar2 are the same as or different from each other, and may be each independently a substituted or unsubstituted aryl group having 6 to 40 carbon atoms; or a substituted or unsubstituted heteroaryl group having 2 to 40 carbon atoms.

In one embodiment of the present application, Ar1 and Ar2 are the same as or different from each other, and may be each independently a substituted or unsubstituted aryl group having 6 to 20 carbon atoms; or a substituted or unsubstituted heteroaryl group having 2 to 20 carbon atoms.

In one embodiment of the present application, Ar1 and Ar2 are the same as or different from each other, and may be each independently a substituted or unsubstituted phenyl group; a substituted or unsubstituted biphenyl group; a substituted or unsubstituted terphenyl group; a substituted or unsubstituted fluorenyl group; or a substituted or unsubstituted naphthyl group.

In one embodiment of the present application, Ar1 and Ar2 are the same as or different from each other, and may be each independently a substituted or unsubstituted phenyl group; a substituted or unsubstituted biphenyl group; a substituted or unsubstituted terphenyl group; a fluorenyl group unsubstituted or substituted with one or more selected from the group consisting of alkyl groups having 1 to 10 carbon atoms; or a substituted or unsubstituted naphthyl group.

In another embodiment, Rp and Rq of Chemical Formula 1 are the same as or different from each other, and may be each independently hydrogen; deuterium; a halogen group; or a substituted or unsubstituted alkyl group having 1 to 60 carbon atoms.

In another embodiment, Rp and Rq are the same as or different from each other, and may be each independently hydrogen; deuterium; a halogen group; or a substituted or unsubstituted alkyl group having 1 to 30 carbon atoms.

In another embodiment, Rp and Rq are the same as or different from each other, and may be each independently hydrogen; deuterium; a halogen group; or a substituted or unsubstituted alkyl group having 1 to 10 carbon atoms.

In another embodiment, Rp and Rq are the same as or different from each other, and may be each independently hydrogen; or deuterium.

In another embodiment, Rp and Rq are hydrogen.

In one embodiment of the present application, p and q of Chemical Formula 1 are each an integer of 0 to 3, and when p and q are each 2 or greater, substituents in the parentheses may be the same as or different from each other.

In another embodiment, p is 0.

In another embodiment, p is 1.

In another embodiment, p is 2.

In another embodiment, p is 3.

In one embodiment of the present application, when p is 2 or greater, substituents in the parentheses may be the same as or different from each other.

In another embodiment, q is 0.

In another embodiment, q is 1.

In another embodiment, q is 2.

In another embodiment, q is 3.

In one embodiment of the present application, when q is 2 or greater, substituents in the parentheses may be the same as or different from each other.

In one embodiment of the present application, Chemical Formula 1 may be represented by any one of the following Chemical Formulae 1-1 to 1-4.

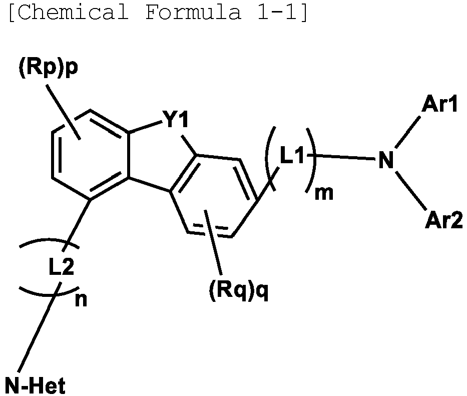

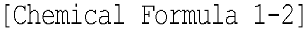

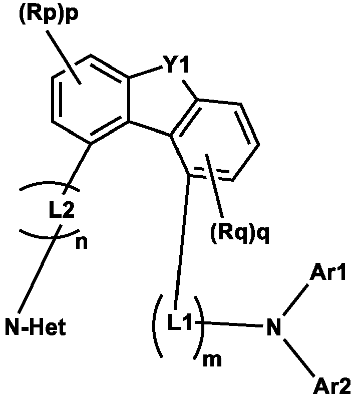

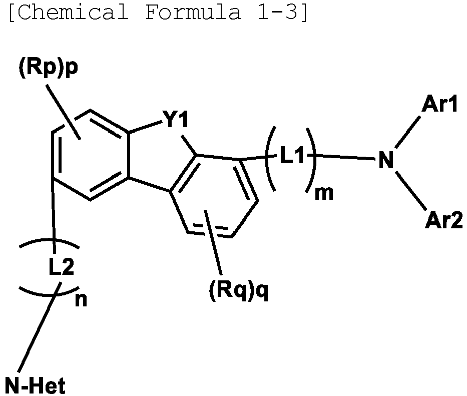

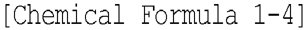

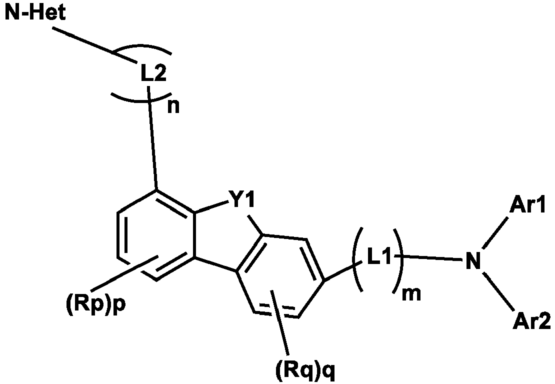

In Chemical Formulae 1-1 to 1-4,
substituents have the same definitions as in Chemical Formula 1.

According to one embodiment of the present application, Chemical Formula 1 may be represented by any one of the following compounds, but is not limited thereto.

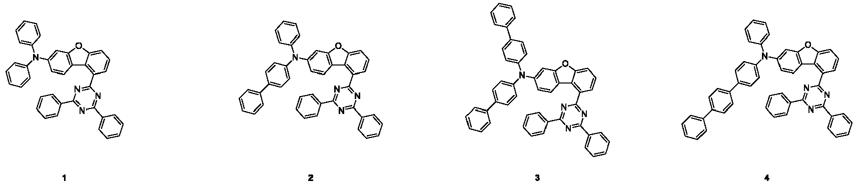

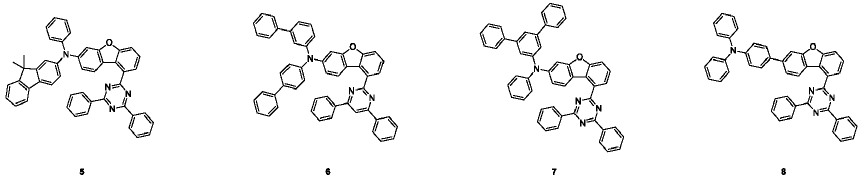

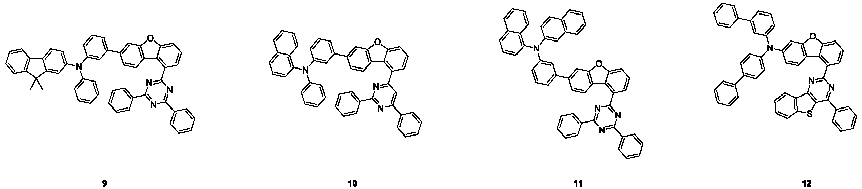

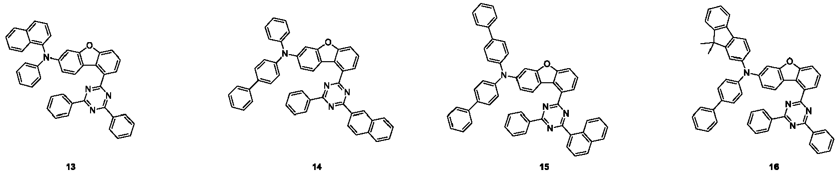

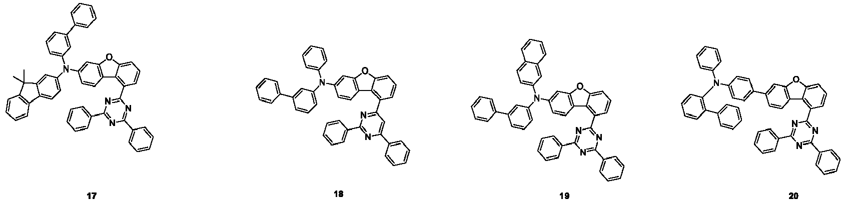

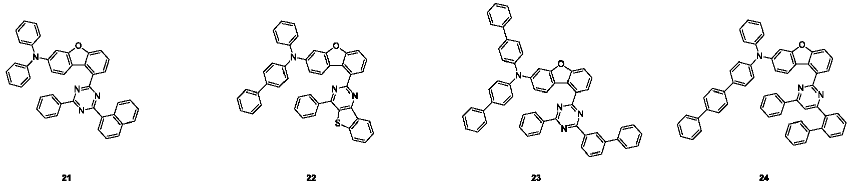

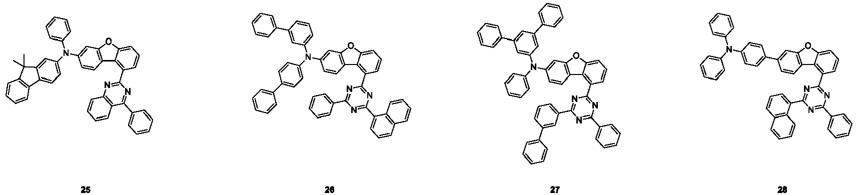

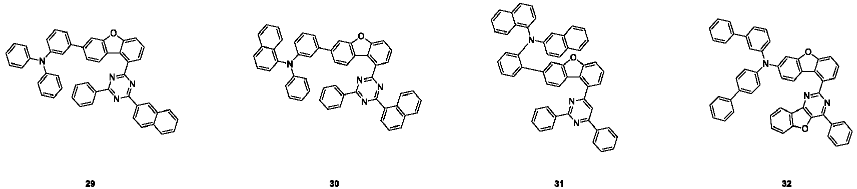

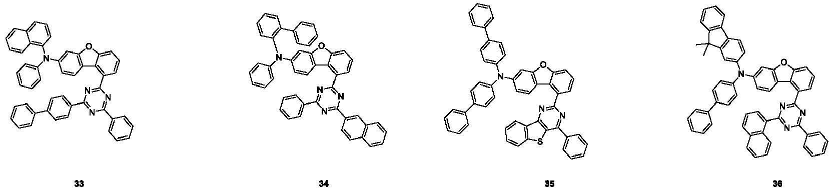

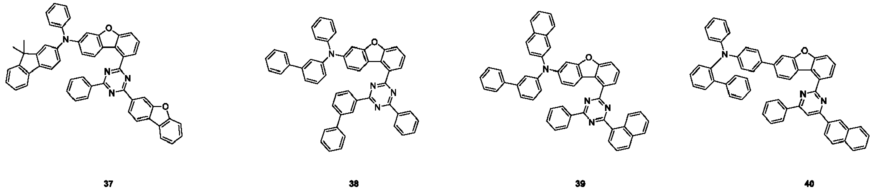

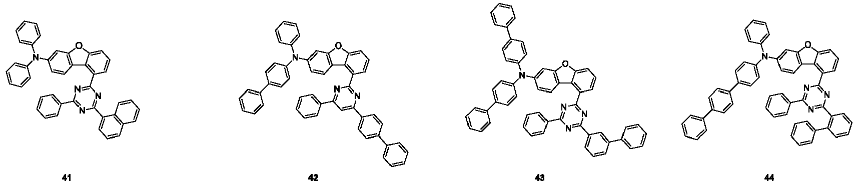

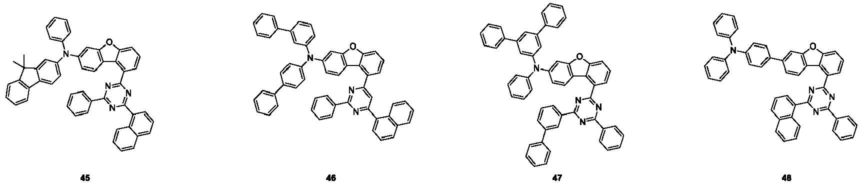

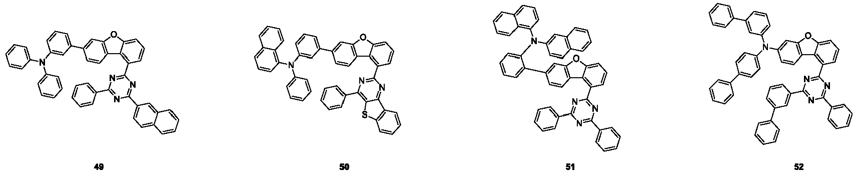

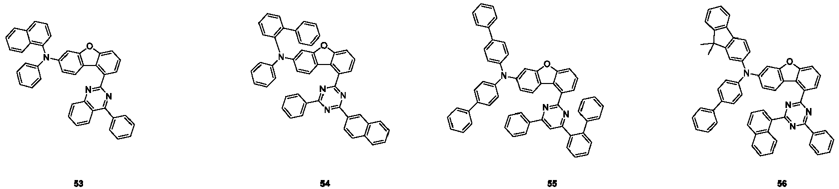

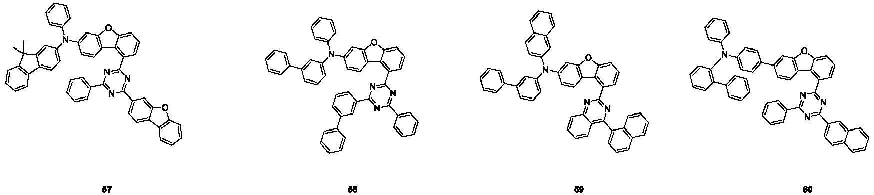

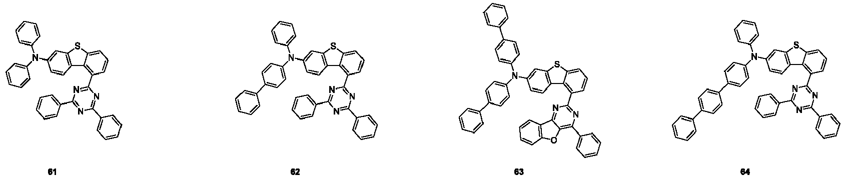

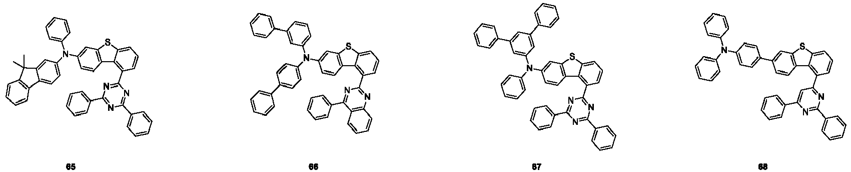

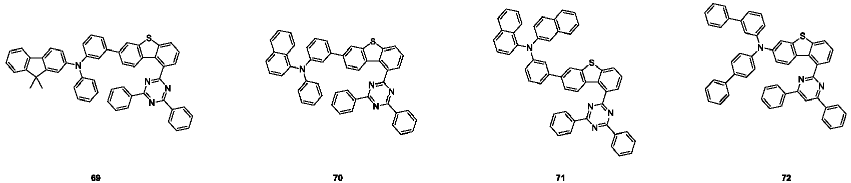

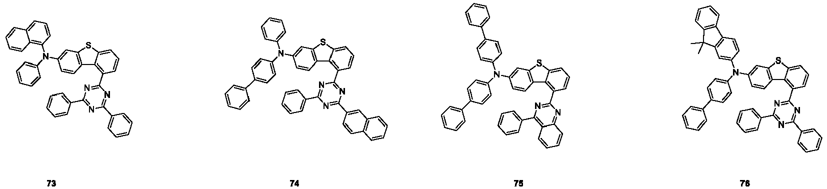

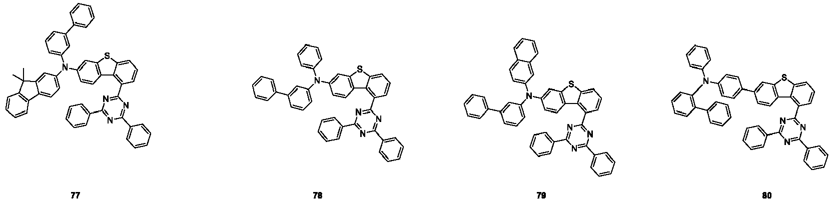

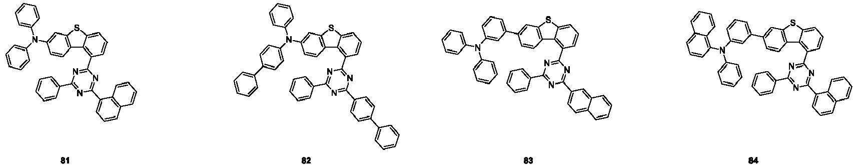

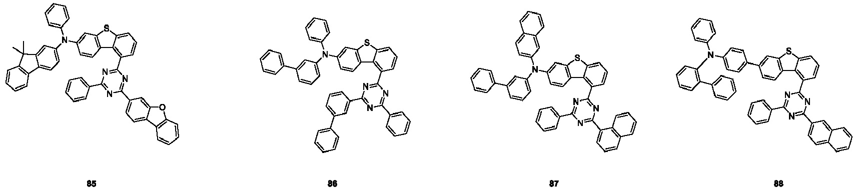

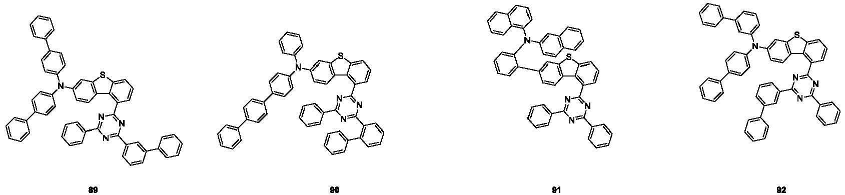

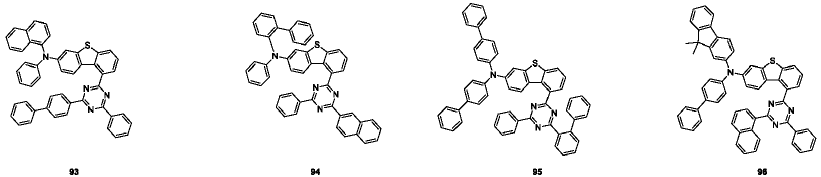

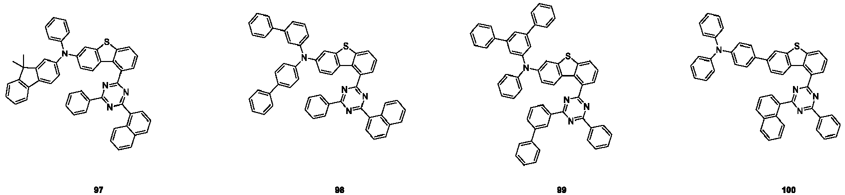

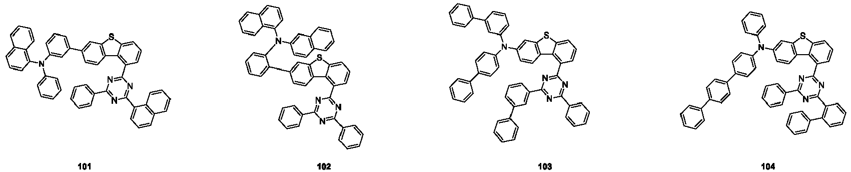

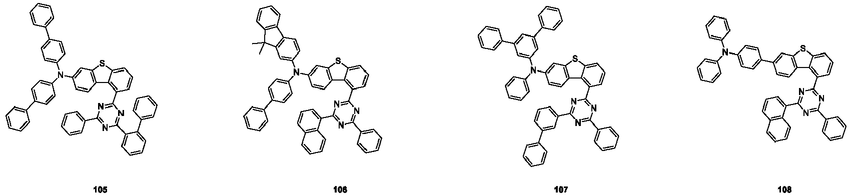

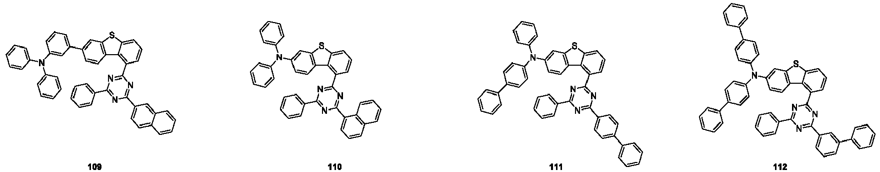

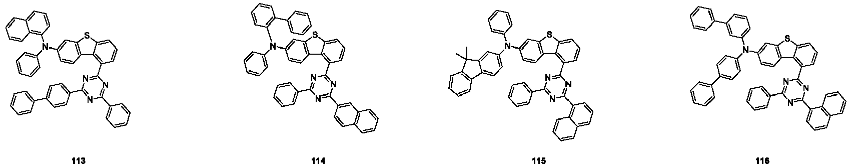

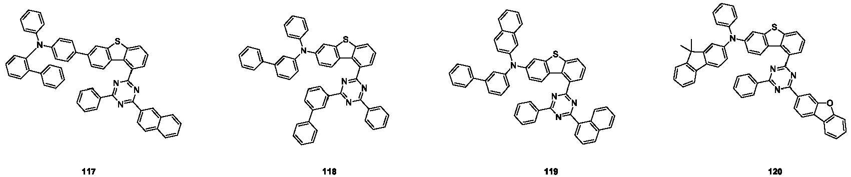

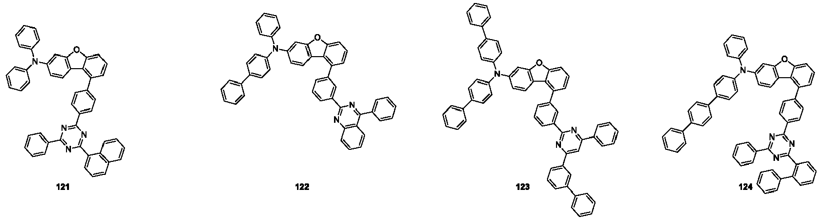

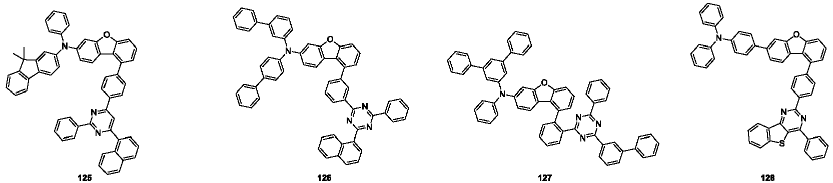

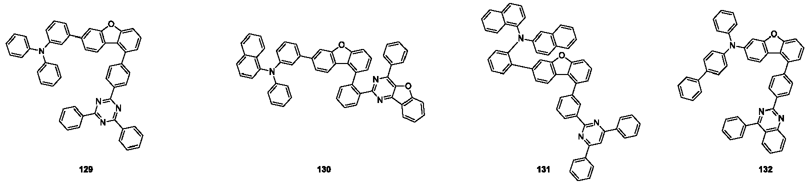

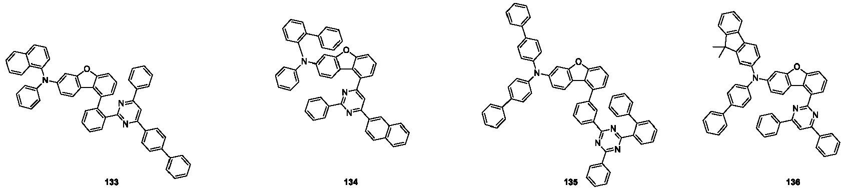

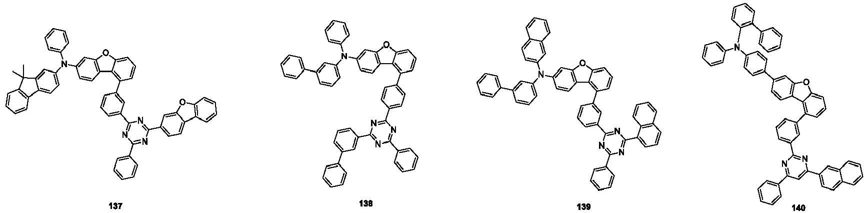

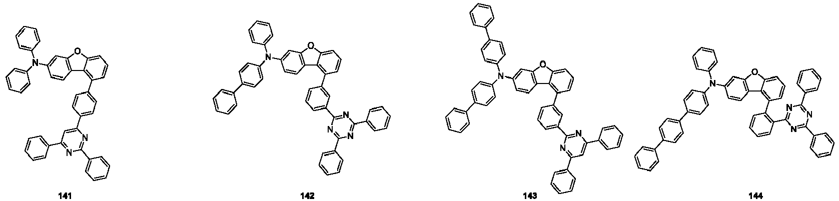

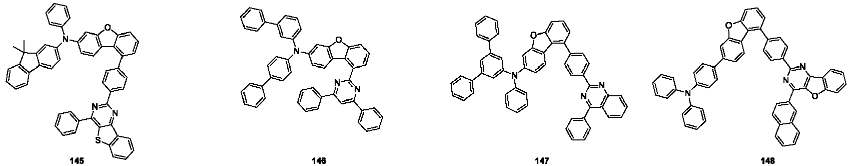

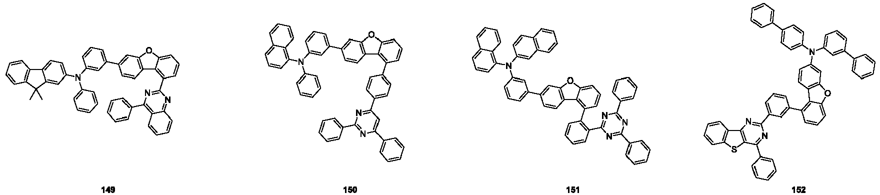

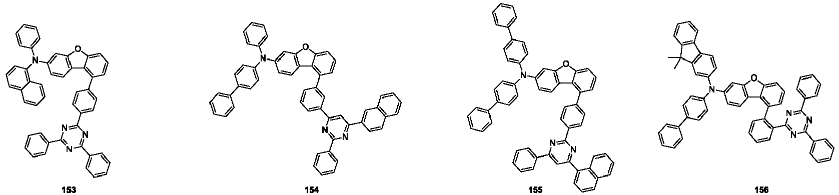

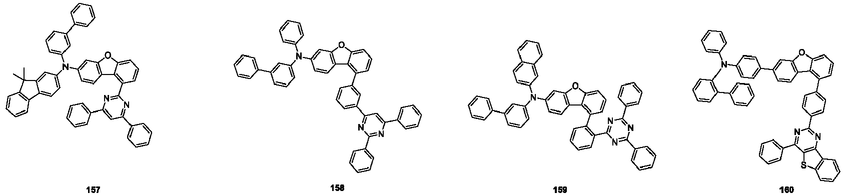

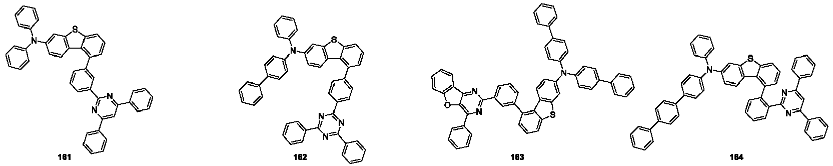

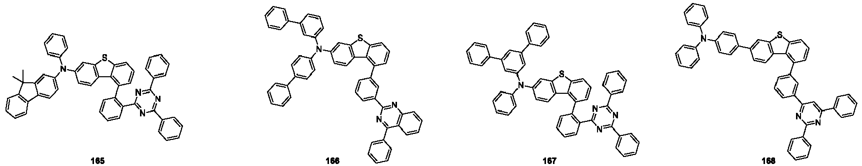

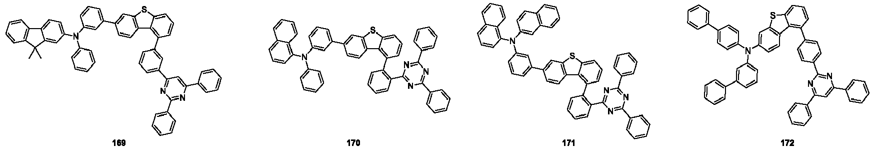

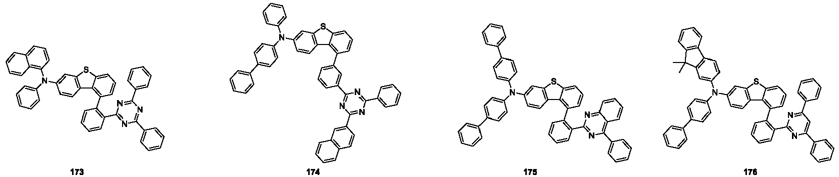

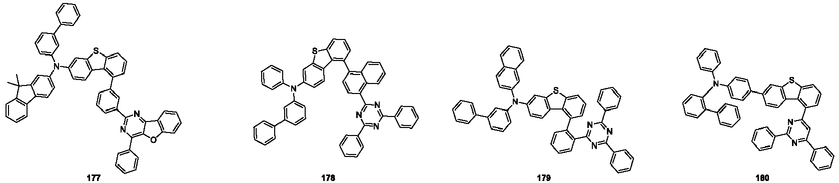

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

Meanwhile, the heterocyclic compound has a high glass transition temperature (Tg), and has excellent thermal stability. Such an increase in the thermal stability becomes an important factor providing driving stability to a device.

The heterocyclic compound according to one embodiment of the present application may be prepared using a multi-step chemical reaction. Some intermediate compounds are prepared first, and from the intermediate compounds, the compound of Chemical Formula 1 may be prepared. More specifically, the heterocyclic compound according to one embodiment of the present application may be prepared based on preparation examples to describe later.

Another embodiment of the present application provides an organic light emitting device comprising the heterocyclic compound represented by Chemical Formula 1. The "organic light emitting device" may be expressed in terms such as an "organic light emitting diode", an "OLED", an "OLED device" and an "organic electroluminescent device".

The heterocyclic compound may be formed into an organic material layer using a solution coating method as well as a vacuum deposition method when manufacturing the organic light emitting device. Herein, the solution coating method means spin coating, dip coating, inkjet printing, screen printing, a spray method, roll coating and the like, but is not limited thereto.

Specifically, the organic light emitting device according to one embodiment of the present application comprises a first electrode, a second electrode, and one or more organic material layers provided between the first electrode and the second electrode, and one or more layers of the organic material layers comprise the heterocyclic compound represented by Chemical Formula 1. When comprising the heterocyclic compound represented by Chemical Formula 1 in the organic material layer, the organic light emitting device has superior light emission efficiency and lifetime.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a red organic light emitting device, and the heterocyclic compound according to Chemical Formula 1 may be used as a material of the red organic light emitting device. The heterocyclic compound according to Chemical Formula 1 has a high HOMO level, and is considered to be more suitable as a red host of an organic light emitting device.

In one embodiment of the present application, the heterocyclic compound according to Chemical Formula 1 may be used as a P-type host.

In the organic light emitting device of the present application, the light emitting layer may further comprise a dopant. As the dopant, materials common in the art may be used, and a material such as (piq)₂(Ir) (acac) may be used.

A content of the dopant may be from 0.5% by weight to 5% by weight, preferably from 1% by weight to 4% by weight, and more preferably from 1.5% by weight to 3.5% by weight based on the whole light emitting layer.

In addition, the organic material layer comprises one or more light emitting layers, and the light emitting layer comprises the heterocyclic compound represented by Chemical Formula 1. When comprising the heterocyclic compound represented by Chemical Formula 1 in the light emitting layer among the organic material layers, the organic light emitting device has more superior light emission efficiency and lifetime.

In addition, in the organic light emitting device of the present application, the organic material layer may further comprise a heterocyclic compound represented by the following Chemical Formula 2.

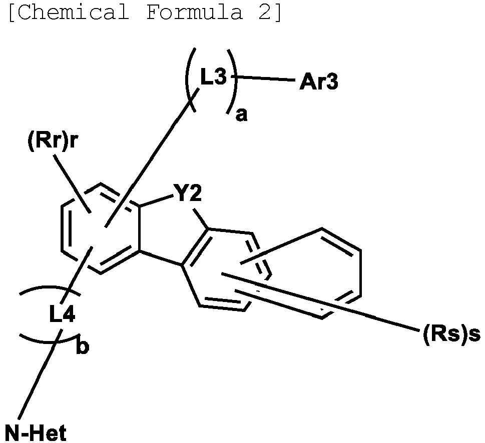

In Chemical Formula 2,
Y2 is O; or S,
L3 and L4 are the same as or different from each other, and each independently a direct bond; a substituted or unsubstituted arylene group having 6 to 60 carbon atoms; or a substituted or unsubstituted heteroarylene group having 2 to 60 carbon atoms, a and b are each an integer of 0 to 3, and when a and b are each 2 or greater, substituents in the parentheses are the same as or different from each other,
N-Het is a monocyclic or polycyclic heterocyclic group substituted or unsubstituted and comprising one or more Ns,
Ar3s are the same as or different from each other, and each independently a substituted or unsubstituted aryl group having 6 to 60 carbon atoms; or a substituted or unsubstituted heteroaryl group having 2 to 60 carbon atoms, and
Rr and Rs are the same as or different from each other, and each independently hydrogen; deuterium; a halogen group; or a substituted or unsubstituted alkyl group having 1 to 60 carbon atoms, r is an integer of 0 to 2, and when r is 2, substituents in the parentheses are the same as or different from each other, s is an integer of 0 to 6, and when s is 2 or greater, substituents in the parentheses are the same as or different from each other.

In one embodiment of the present application, Y2 of Chemical Formula 2 may be O; or S.

In another embodiment, Y2 is O.

In another embodiment, Y2 is S.

In one embodiment of the present application, L3 and L4 of Chemical Formula 2 are the same as or different from each other, and each independently a direct bond; a substituted or unsubstituted arylene group having 6 to 60 carbon atoms; or a substituted or unsubstituted heteroarylene group having 2 to 60 carbon atoms.

In one embodiment of the present application, L3 and L4 are the same as or different from each other, and may be each independently a direct bond; a substituted or unsubstituted arylene group having 6 to 40 carbon atoms; or a substituted or unsubstituted heteroarylene group having 2 to 40 carbon atoms.

In one embodiment of the present application, L3 and L4 are the same as or different from each other, and may be each independently a direct bond; a substituted or unsubstituted arylene group having 6 to 20 carbon atoms; or a substituted or unsubstituted heteroarylene group having 2 to 20 carbon atoms.

In one embodiment of the present application, L3 may be a direct bond; a substituted or unsubstituted arylene group having 6 to 60 carbon atoms; or a substituted or unsubstituted heteroarylene group having 2 to 60 carbon atoms.

In another embodiment, L3 is a direct bond.

In another embodiment, L3 is an arylene group having 6 to 60 carbon atoms; or a substituted or unsubstituted heteroarylene group having 2 to 60 carbon atoms.

In another embodiment, L3 is a phenylene group.

In another embodiment, L3 is a naphthylene group.

In one embodiment of the present application, L4 may be a direct bond; a substituted or unsubstituted arylene group having 6 to 60 carbon atoms; or a substituted or unsubstituted heteroarylene group having 2 to 60 carbon atoms.

In another embodiment, L4 is a direct bond.

In another embodiment, L4 is an arylene group having 6 to 60 carbon atoms; or a substituted or unsubstituted heteroarylene group having 2 to 60 carbon atoms.

In another embodiment, L4 is a phenylene group.

In one embodiment of the present application, a and b of Chemical Formula 2 are each an integer of 0 to 3, and when a and b are each 2 or greater, substituents in the parentheses may be the same as or different from each other.

In another embodiment, a is 0.

In another embodiment, a is 1.

In another embodiment, a is 2.

In another embodiment, a is 3.

In one embodiment of the present application, when a is 2 or greater, substituents in the parentheses may be the same as or different from each other.

In another embodiment, b is 0.

In another embodiment, b is 1.

In another embodiment, b is 2.

In another embodiment, b is 3.

In one embodiment of the present application, when b is 2 or greater, substituents in the parentheses may be the same as or different from each other.

In one embodiment of the present application, N-Het of Chemical Formula 2 has the same definition as in Chemical Formula 1.

In one embodiment of the present application, Ar3 of Chemical Formula 2 may be a substituted or unsubstituted aryl group having 6 to 60 carbon atoms; or a substituted or unsubstituted heteroaryl group having 2 to 60 carbon atoms.

In one embodiment of the present application, Ar3s are the same as or different from each other, and may be each independently a substituted or unsubstituted aryl group having 6 to 40 carbon atoms; or a substituted or unsubstituted heteroaryl group having 2 to 40 carbon atoms.

In one embodiment of the present application, Ar3s are the same as or different from each other, and may be each independently a substituted or unsubstituted aryl group having 6 to 40 carbon atoms; or a substituted or unsubstituted heteroaryl group having 2 to 40 carbon atoms.

In one embodiment of the present application, Ar3 may be a substituted or unsubstituted phenyl group; a substituted or unsubstituted biphenyl group; a substituted or unsubstituted fluorenyl group; or a substituted or unsubstituted naphthyl group.

In one embodiment of the present application, Ar3 may be a substituted or unsubstituted phenyl group; a substituted or unsubstituted biphenyl group; a fluorenyl group unsubstituted or substituted with one or more selected from the group consisting of alkyl groups having 1 to 10 carbon atoms; or a substituted or unsubstituted naphthyl group.

In one embodiment of the present application, Rr and Rs of Chemical Formula 2 are hydrogen.

According to one embodiment of the present application, Chemical Formula 2 may be represented by any one of the following Chemical Formulae 2-1 to 2-3.

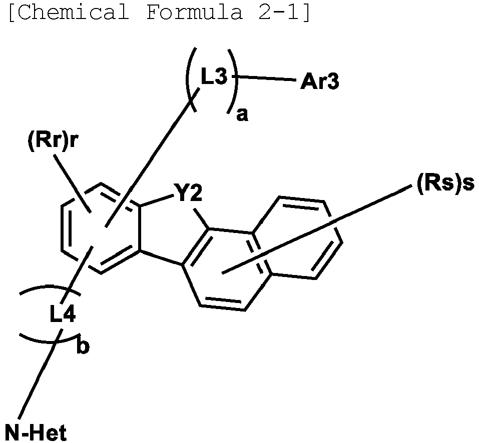

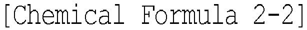

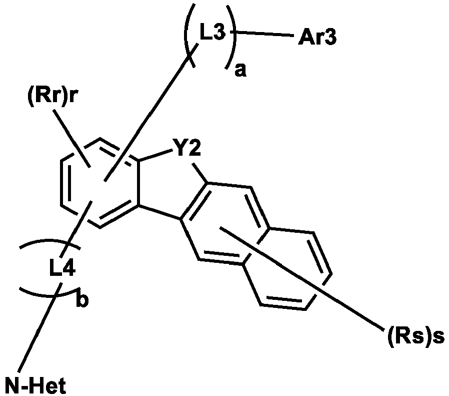

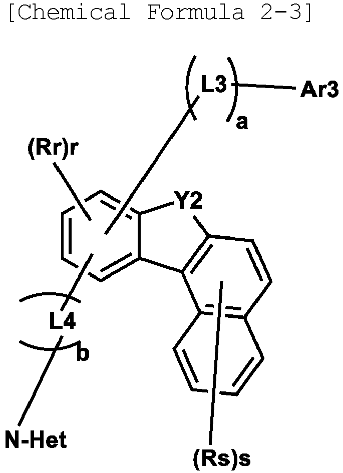

In Chemical Formulae 2-1 to 2-3,
substituents have the same definitions as in Chemical Formula 2.

According to one embodiment of the present application, Chemical Formula 2 may be represented by any one of the following compounds, but is not limited thereto.

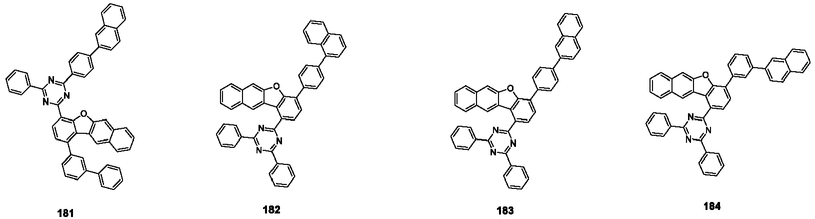

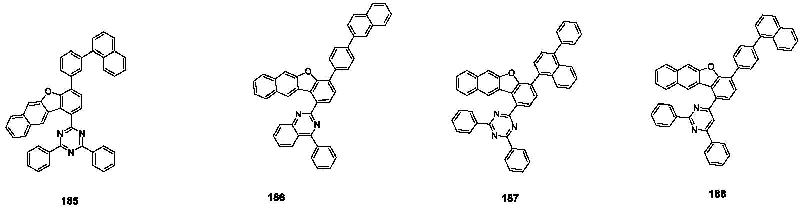

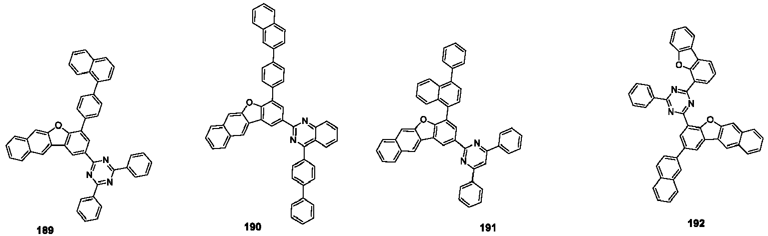

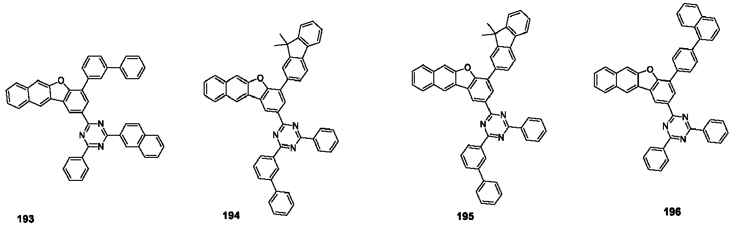

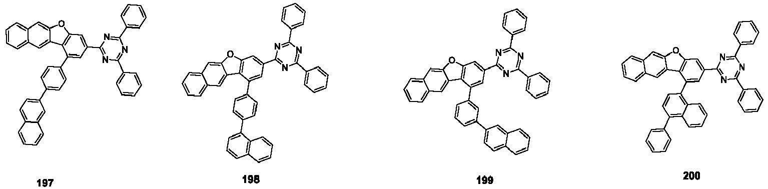

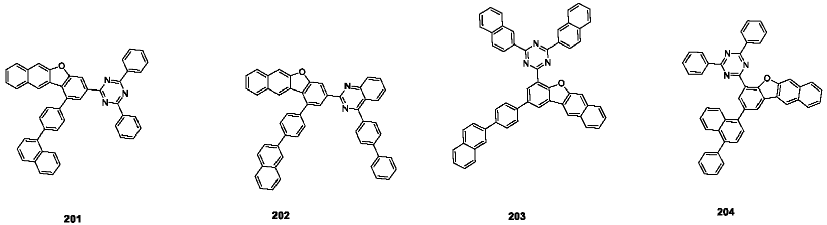

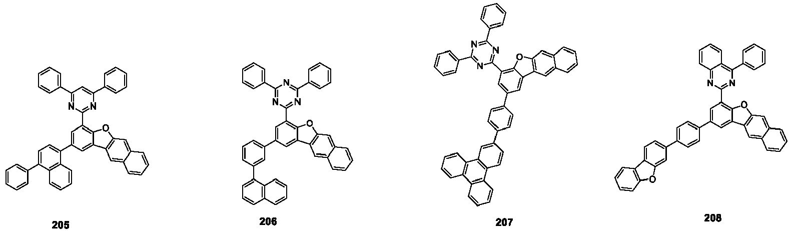

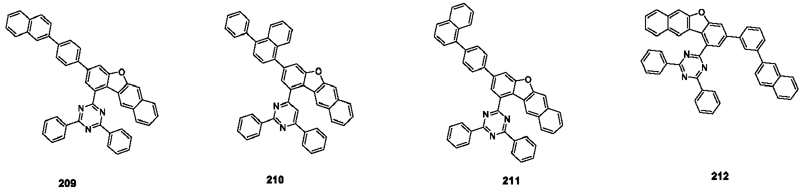

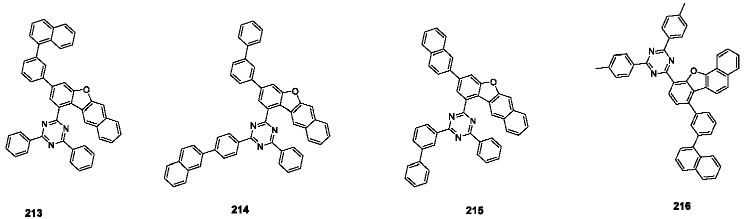

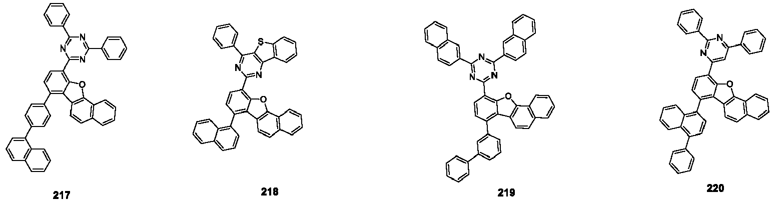

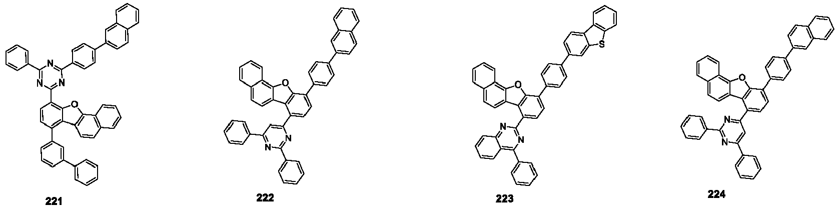

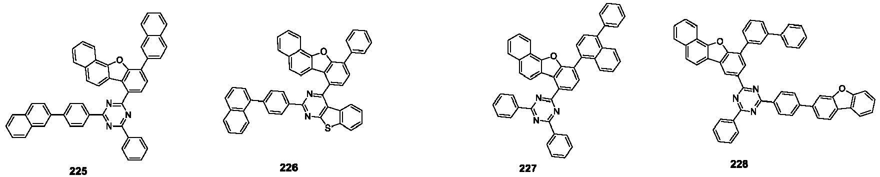

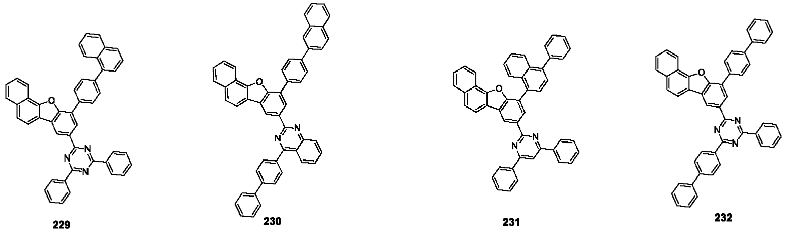

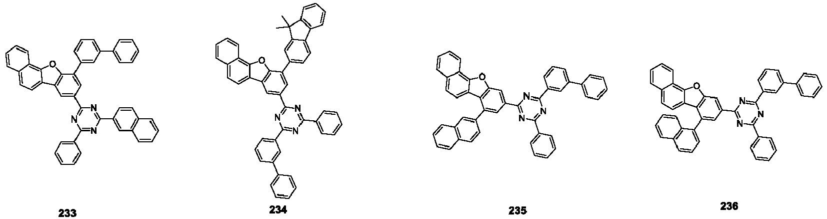

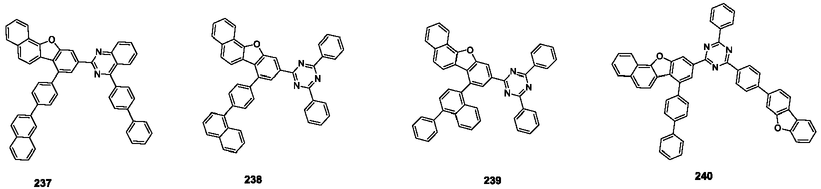

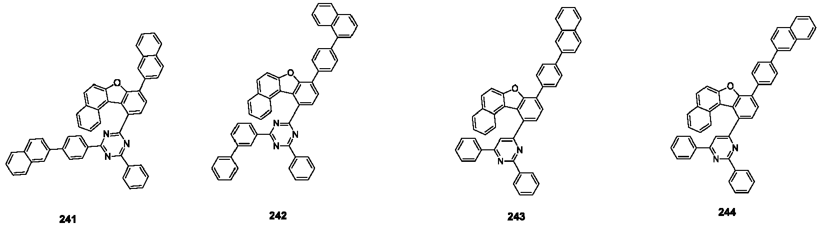

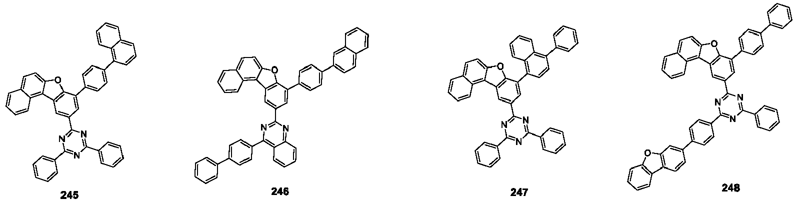

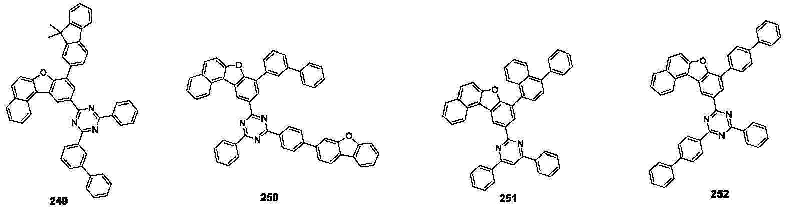

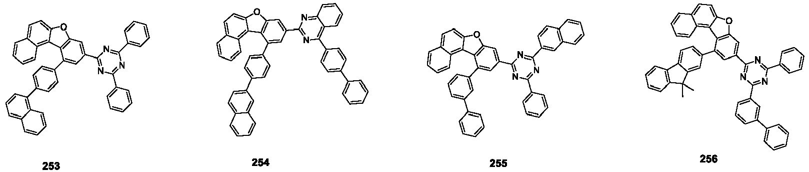

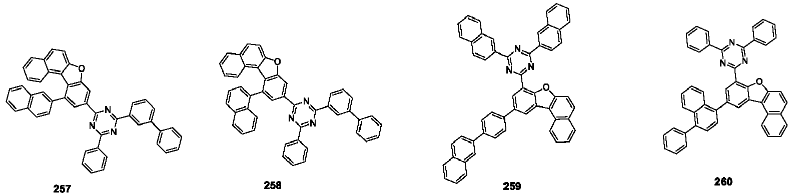

In addition, the organic material layer comprises one or more light emitting layers, and the light emitting layer comprises the heterocyclic compound represented by Chemical Formula 1 as a first compound, and further comprises the heterocyclic compound represented by Chemical Formula 2 as a second compound. When comprising the heterocyclic compound represented by Chemical Formula 1 in the light emitting layer among the organic material layers, the organic light emitting device has more superior light emission efficiency and lifetime.

In one embodiment of the present application, the heterocyclic compound according to Chemical Formula 2 may be used as an N-type host.

As a result, it may be expected that an exciplex phenomenon occurs when comprising the heterocyclic compound represented by Chemical Formula 1 according to the present application corresponding to a P-type host compound having strong hole transfer properties and the heterocyclic compound represented by Chemical Formula 2 according to the present application corresponding to an N-type host compound at the same time.

In addition, by introducing various substituents to the structure of Chemical Formula 2, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 2, the energy band gap may be finely controlled, and meanwhile, properties at interfaces between organic materials are enhanced, and material applications may become diverse.

Meanwhile, the heterocyclic compound has a high glass transition temperature (Tg), and has excellent thermal stability. Such an increase in the thermal stability becomes an important factor providing driving stability to a device.

The heterocyclic compound according to one embodiment of the present application may be prepared using a multi-step chemical reaction. Some intermediate compounds are prepared first, and from the intermediate compounds, the compound of Chemical Formula 2 may be prepared. More specifically, the heterocyclic compound according to one embodiment of the present application may be prepared based on preparation examples to describe later.

In addition, the organic material layer comprises one or more light emitting layers, and the light emitting layer comprises the heterocyclic compound represented by Chemical Formula 1 and the heterocyclic compound represented by Chemical Formula 2 at the same time. When comprising the heterocyclic compound represented by Chemical Formula 1 and the heterocyclic compound represented by Chemical Formula 2 in the light emitting layer among the organic material layers, the organic light emitting device has more superior light emission efficiency and lifetime due to an exciplex phenomenon.

In the organic light emitting device of the present application, the organic material layer comprises a light emitting layer, and the light emitting layer may comprise the heterocyclic compound as a host material of a light emitting material.

In the organic light emitting device of the present application, the light emitting layer may comprise two or more host materials, and at least one of the host materials may comprise the heterocyclic compound as a host material of a light emitting material.

In the organic light emitting device of the present application, two or more host materials may be pre-mixed and used as the light emitting layer, and at least one of the two or more host materials may comprise the heterocyclic compound as a host material of a light emitting material.

The pre-mixing means placing and mixing two or more host materials of the light emitting layer in one source of supply before depositing on the organic material layer.

In the organic light emitting device of the present application, the light emitting layer may comprise two or more host materials, and the two or more host materials may each comprise one or more p-type host materials and n-type host materials, and at least one of the host materials may comprise the heterocyclic compound as a host material of a light emitting material. In this case, the organic light emitting device may have superior driving, efficiency and lifetime.

In the organic light emitting device of the present application, the light emitting layer may further comprise a dopant even when comprising two or more host materials. As the dopant, materials common in the art may be used, and a material such as (piq)₂(Ir) (acac) may be used.

A content of the dopant may be from 0.5% by weight to 5% by weight, preferably from 1% by weight to 4% by weight, and more preferably from 1.5% by weight to 3.5% by weight based on the whole light emitting layer.

The organic light emitting device of the present disclosure may further comprise one, two or more layers selected from the group consisting of a light emitting layer, a hole injection layer, a hole transfer layer, an electron injection layer, an electron transfer layer, a hole auxiliary layer and a hole blocking layer.

The organic light emitting device according to one embodiment of the present application may be manufactured using common organic light emitting device manufacturing methods and materials except that the organic material layer is formed using the heterocyclic compound described above.

In addition, another embodiment of the present application provides a composition for an organic material layer of an organic light emitting device, the composition comprising the heterocyclic compound represented by Chemical Formula 1 and the heterocyclic compound represented by Chemical Formula 2 at the same time.

In another embodiment of the present application, the heterocyclic compound represented by Chemical Formula 1:the heterocyclic compound represented by Chemical Formula 2 may have a weight ratio of 1:10 to 10:1, 1:8 to 8:1, 1:5 to 5:1 or 1:2 to 2:1 in the composition, however, the weight ratio is not limited thereto.

Specific descriptions on the heterocyclic compound represented by Chemical Formula 1 and the compound represented by Chemical Formula 2 that the composition for an organic material layer comprises are the same as the descriptions provided above.

FIG. 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

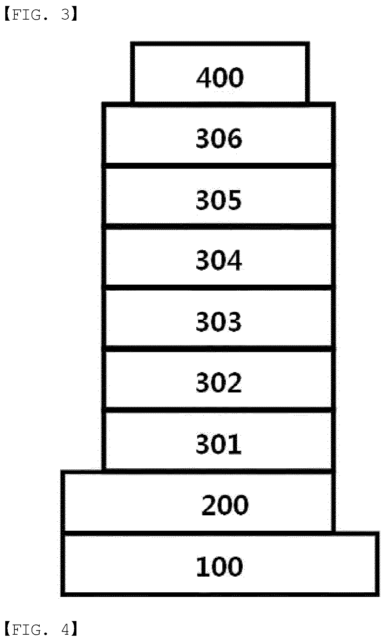

FIG. 3 illustrates a case of the organic material layer being a multilayer. The organic light emitting device according to FIG. 3 comprises a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be comprised, and other necessary functional layers may be further added.

In addition, the organic light emitting device according to one embodiment of the present application comprises a first electrode; a first stack provided on the first electrode and comprising a first light emitting layer; a charge generation layer provided on the first stack; a second stack provided on the charge generation layer and comprising a second light emitting layer; and a second electrode provided on the second stack.

Herein, the charge generation layer may comprise the heterocyclic compound represented by Chemical Formula 1. When the heterocyclic compound is used in the charge generation layer, the organic light emitting device may have superior driving, efficiency and lifetime.

In addition, the first stack and the second stack may each independently further comprise one or more types of the hole injection layer, the hole transfer layer, the hole blocking layer, the electron transfer layer, the electron injection layer and the like described above.

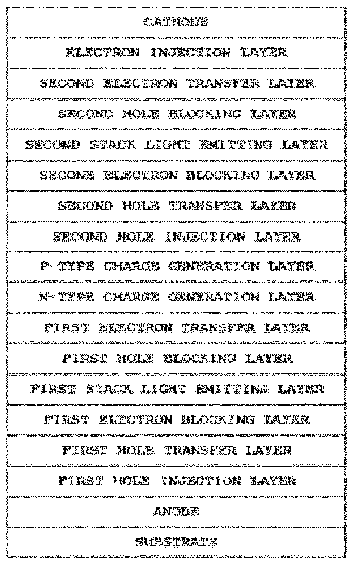

As the organic light emitting device according to one embodiment of the present application, an organic light emitting device having a 2-stack tandem structure is schematically illustrated in FIG. 4.

Herein, the first electron blocking layer, the first hole blocking layer, the second hole blocking layer and the like described in FIG. 4 may not be comprised in some cases.

In the organic light emitting device according to one embodiment of the present application, materials other than the heterocyclic compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material comprise metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material comprise metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

The organic light emitting device according to one embodiment of the present application may be a top-emission type, a bottom-emission type or a dual-emission type depending on the materials used.

The heterocyclic compound according to one embodiment of the present application may also be used in an organic electronic device comprising an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example 1> Preparation of Compound 3

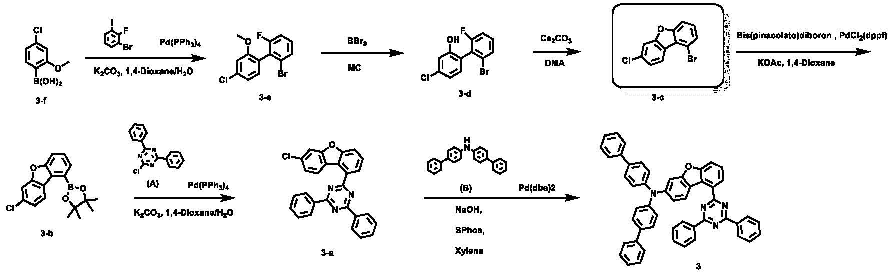

### 1) Preparation of Compound 3-e

In a one neck round bottom flask (one neck r.b.f), a mixture of (4-chloro-2-methoxyphenyl)boronic acid (Compound 3-f) (30.98 g, 166.21 mmol), 1-bromo-2-fluoro-3-iodobenzene (50 g, 151.10 mmol), tetrakis(triphenylphosphine)palladium(0) (8.73 g, 7.56 mmol), potassium carbonate (41.77 g, 302.2 mmol) and 1,4-dioxane/H₂O (500 ml/150 ml) was refluxed at 120°C. After that, the result was extracted with dichloromethane, and dried with MgSO₄. After the drying, the result was column purified to obtain Compound 3-e (34.8 g, 730).

### 2) Preparation of Compound 3-d

In a one neck round bottom flask (one neck r.b.f), a mixture of 2'-bromo-4-chloro-6'-fluoro-2-methoxy-1,1'-biphenyl (Compound 3-e) (34.8 g, 110.28 mmol) and methylene chloride (MC) (348 ml) was cooled to 0°C, and after adding BBr₃ (20.9 ml, 220.56 mmol) dropwise thereto, the temperature was raised to room temperature, and the mixture was stirred for 2 hours. The reaction was terminated using distilled water, and the result was extracted with dichloromethane and dried with MgSO₄. After the drying, the result was silica gel filtered, and then concentrated to obtain Compound 3-d (33.25 g, 1000).

### 3) Preparation of Compound 3-c

In a one neck round bottom flask (one neck r.b.f), a mixture of 2'-bromo-4-chloro-6'-fluoro-[1,1'-biphenyl]-2-ol (Compound 3-d) (33.25 g, 110.27 mmol), Cs₂CO₃ (71.86 g, 220.54 mmol) and dimethylacetamide (330 ml) was stirred at 150°C. The result was cooled and then filtered, and the solvent of the filtrate was silica gel filtered to obtain Compound 3-c (24.84 g, 80%).

### 4) Preparation of Compound 3-b

In a one neck round bottom flask (one neck r.b.f), a mixture of 1-bromo-7-chlorodibenzo[b,d]furan (Compound 3-c) (24.84 g, 88.23 mmol), bis(pinacolato)diboron (44.81 g, 176.46 mmol), potassium acetate (25.98 g, 264.69 mmol), PdCl₂(dppf) (3.23 g, 4.41 mmol) and 1,4-dioxane (240 ml) was refluxed at 140°C. After that, the result was extracted with dichloromethane, concentrated, and then silica gel filtered to obtain Compound 3-b (34.79 g, over yield).

### 5) Preparation of Compound 3-a

In a one neck round bottom flask (one neck r.b.f), a mixture of 2-(7-chlorodibenzo[b,d]furan-1-yl)-4,4,5,5-tetramethyl-1,3,2-dioxaborolane (Compound 3-d) (34.79 g, 105.87 mmol), 2-chloro-4,6-diphenyl-1,3,5-triazine (A) (31.18 g, 116.46 mmol), tetrakis(triphenylphosphine)palladium(0) (6.12 g, 5.29 mmol), potassium carbonate (29.26 g, 211.74 mmol) and 1,4-dioxane/water (340 ml/102 ml) was refluxed for 3 hours at 120°C. After that, the result was filtered at room temperature (RT), and then washed with distilled water and acetone to obtain Compound 3-a (44.56 g, 97%).

### 6) Preparation of Target Compound 3

In a one neck round bottom flask (one neck r.b.f), a mixture of 2-(7-chlorodibenzo[b,d]furan-1-yl)-4,6-diphenyl-1,3,5-triazine (Compound 3-a) (44.56 g, 102.70 mmol), di([1,1'-biphenyl]-4-yl)amine (B) (36.31 g, 112.97 mmol), Pd(dba)₂ (5.95 g, 5.14 mmol), sodium hydroxide (8.22 g, 205.4 mmol), SPhos (6.32 g, 15.41 mmol) and xylene (445 ml) was refluxed for 8 hours (h) at 180°C. The result was cooled and then filtered, and washed with distilled water and acetone to obtain target Compound 3 (61.28 g, 83%).

Target compounds of the following Table 1 were prepared in the same manner as in Preparation of target Compound 3 of Preparation Example 1 except that Intermediates A and B of the following Table 1 were used instead of 2-chloro-4,6-diphenyl-1,3,5-triazine (A) and di([1,1'-biphenyl]-4-yl)amine (B), respectively.

**[Table 1]**

| Compound | Intermediate A | Intermediate B | Target Compound | Yield |
|---|---|---|---|---|
| 3 | 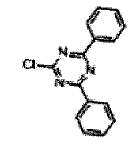 | 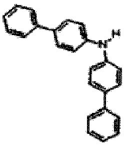 | 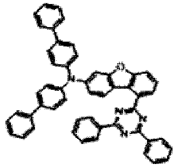 | 79% |
| 8 | 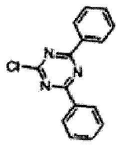 | 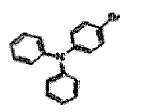 | 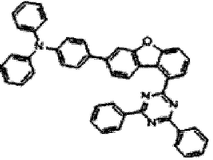 | 60% |
| 19 | 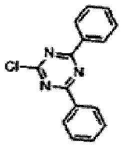 | 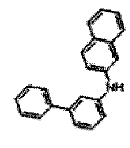 | 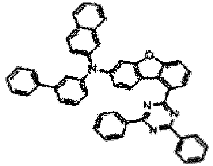 | 88% |
| 46 | 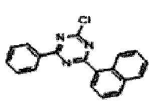 | 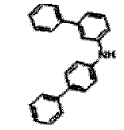 | 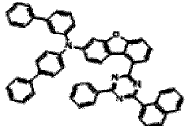 | 76% |
| 56 | 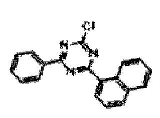 | 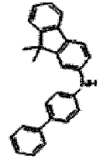 | 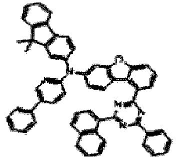 | 91% |
| 124 | 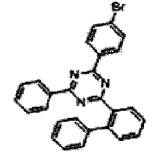 | 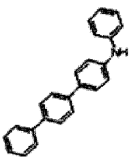 | 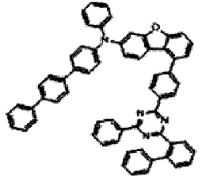 | 65% |
| 127 | 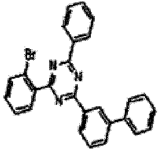 | 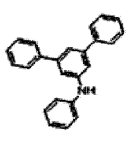 | 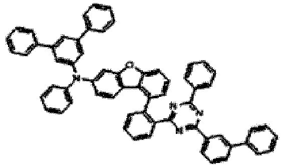 | 88% |
| 152 | 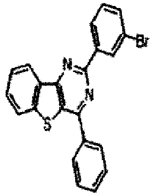 | 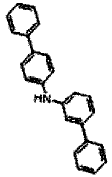 | 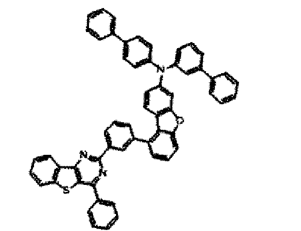 | 69% |
| 157 | 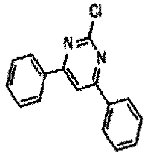 | 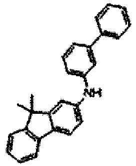 | 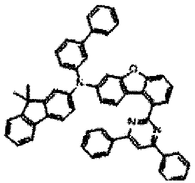 | 81% |

Compounds other than the compounds of Preparation Example 1 were all synthesized in the same manner as in Preparation Example 1, and the synthesis identification results are shown in the following Table 2 and Table 3. The following Table 2 shows measurement values of FD-mass spectrometry (FD-MS: field desorption mass spectrometry), and the following Table 3 shows measurement values of ¹H NMR (CDCl₃, 300 Mz) .

**[Table 2]**

| Comp ound | FD-MS | Compo und | FD-MS |
|---|---|---|---|
| 1 | m/z=566.21 (C₃₉H₂₆N₄O=566.65) | 2 | m/z=642.24 (C₄₅H₃₀N₄O=642.75) |
| 3 | m/z=718.27 (C₅₁H₃₄N₄O=718.84) | 4 | m/z=718.27 (C₅₁H₃₄N₄O=718.84) |
| 5 | m/z=682.27 (C₄₈H₃₄N₄O=682.81) | 6 | m/z=717.28 (C₅₂H₃₄N₃O=717.85) |
| 7 | m/z=718.27 (C₅₁H₃₄N₄O=718.84) | 8 | m/z=642.24 (C₄₅H₃₀N₄O=642.75) |
| 9 | m/z=758.30 (C₅₄H₃₈N₄O=758.91) | 10 | m/z=691.26 (C₅₀H₃₂N₃O=691.82) |
| 11 | m/z=742.27 (C₅₃H₃₄N₄O=742.86) | 12 | m/z=718.27 (C₅₁H₃₄N₄O=718.84) |
| 13 | m/z=616.23 (C₄₃H₂₈N₄O=616.71) | 14 | m/z=692.26 (C₄₉H₃₂N₄O=692.80) |
| 15 | m/z=768.29 (C₅₅H₃₆N₄O=768.90) | 16 | m/z=758.30 (C₅₄H₃₈N₄O=758.91) |
| 17 | m/z=758.91 (C₅₄H₃₈N₄O=758.30) | 18 | m/z=641.25 (C₄₅H₃₀N₄O=641.76) |
| 19 | m/z=692.26 (C₄₉H₃₂N₄O=692.80) | 20 | m/z=718.27 (C₅₁H₃₄N₄O=718.84) |
| 21 | m/z=616.23 (C₄₈H₂₈N₄O=616.71) | 22 | m/z=718.27 (C₅₁H₃₄N₄O=718.84) |
| 23 | m/z=794.30 (C₅₇H₃₈N₄O=794.94) | 24 | m/z=793.31 (C₅₈H₃₉N₃O=793.95) |
| 25 | m/z=655.26 (C₄₇H₃₃N₃O=655.78) | 26 | m/z=768.29 (C₅₅H₃₆N₄O=768.90) |
| 27 | m/z=794.30 (C₅₇H₃₈N₄O=794.94) | 28 | m/z=692.26 (C₄₉H₃₂N₄O=692.80) |
| 29 | m/z=692.26 (C₄₉H₃₂N₄O=692.80) | 30 | m/z=742.27 (C₅₃H₃₄N₄O=742.86) |
| 31 | m/z=741.28 (C₅₄H₃₅N₃O=741.88) | 32 | m/z=794.30 (C₅₇H₃₈N₄O=794.94) |
| 33 | m/z=692.26 (C₄₉H₃₂N₄O=692.80) | 34 | m/z=692.26 (C₄₉H₃₂N₄O=692.80) |
| 35 | m/z=747.23 (C₅₂H₃₃N₃OS=794.90) | 36 | m/z=808.32 (C₅₈H₄₀N₄O=808.96) |
| 37 | m/z=772.28 (C₅₄H₃₆N₄O₂=772.89) | 38 | m/z=718.27 (C₅₁H₃₄N₄O=718.84) |
| 39 | m/z=742.27 (C₅₃H₃₄N₄O=742.86) | 40 | m/z=767.29 (C₅₆H₃₇N₃O=767.91) |
| 41 | m/z=616.23 (C₄₃H₂₈N₄O=616.71) | 42 | m/z=717.28 (C₅₂H₃₄N₃O=717.85) |
| 43 | m/z=794.30 (C₅₇H₃₈N₄O=794.94) | 44 | m/z=794.30 (C₅₇H₃₈N₄O=794.94) |
| 45 | m/z=732.29 (C₅₂H₃₆N₄O=732.87) | 46 | m/z=768.29 (C₅₅H₃₆N₄O=768.90) |
| 47 | m/z=794.30 (C₅₇H₃₈N₄O=794.94) | 48 | m/z=692.26 (C₄₉H₃₂N₄O=692.80) |
| 49 | m/z=692.26 (C₄₉H₃₂N₄O=692.80) | 50 | m/z=721.22 (C₅₀H₃₁N₃OS=721.87) |
| 51 | m/z=742.27 (C₅₃H₃₄N₄O=742.86) | 52 | m/z=794.30 (C₅₇H₃₈N₄O=794.94) |
| 53 | m/z=589.22 (C₄₂H₂₇N₃O=589.68) | 54 | m/z=692.26 (C₄₉H₃₂N₄O=692.80) |
| 55 | m/z=793.31 (C₅₈H₃₉N₃O=793.95) | 56 | m/z=808.32 (C₅₈H₄₀N₄O=808.96) |
| 57 | m/z=772.28 (C₅₄H₃₆N₄O₂=772.89) | 58 | m/z=718.27 (C₅₁H₃₄N₄O=718.84) |
| 59 | m/z=715.26 (C₅₂H₃₃N₃O=715.84) | 60 | m/z=768.29 (C₅₅H₃₆N₄O=768.90) |
| 61 | m/z=582.19 (C₃₉H₂₆N₄S=582.72) | 62 | m/z=658.22 (C₄₅H₃₀N₄S=658.81) |
| 63 | m/z=707 .24 (C₅₀H₃₃N₃S=707.88) | 64 | m/z=734.25 (C₅₁H₃₄N₄S=734.91) |
| 65 | m/z=698.25 (C₄₈H₃₄N₄S=698.88) | 66 | m/z=707.24 (C₅₀H₃₃N₃S=707.88) |
| 67 | m/z=734.25 (C₅₁H₃₄N₄S=734.91) | 68 | m/z=657.22 (C₄₆H₃₁N₃S=657.82) |
| 69 | m/z=774.28 (C₅₄H₃₈N₄S=774.97) | 70 | m/z=708.23 (C₄₉H₃₂N₄S=708.87) |
| 71 | m/z=758.25 (C₅₃H₃₄N₄S=758.93) | 72 | m/z=733.26 (C₅₂H₃₅N₃S=733.92) |
| 73 | m/ z=632 .20 (C₄₃H₂₈N₄S=632.77) | 74 | m/z=708.23 (C₄₉H₃₂N₄S=708.87) |
| 75 | m/z=784.27 (C₅₅H₃₆N₄S=784.97) | 76 | m/z=774.28 (C₅₄H₃₈N₄S=774.97) |
| 77 | m/z=774.28 (C₅₄H₃₈N₄S=774.97) | 78 | m/z=658.22 (C₄₅H₃₀N₄S=658.81) |
| 79 | m/z=708.23 (C₄₉H₃₂N₄S=708.87) | 80 | m/z=734.25 (C₅₁H₃₄N₄S=734.91) |
| 81 | m/ z=632 .20 (C₄₃H₂₈N₄S=632.77) | 82 | m/z=734.25 (C₅₁H₃₄N₄S=734.91) |
| 83 | m/z=708.23 (C₄₉H₃₂N₄S=708.87) | 84 | m/z=758.25 (C₅₃H₃₄N₄S=758.93) |
| 85 | m/z=788.26 (C₅₄H₃₆N₄OS=788.95) | 86 | m/z=734.25 (C₅₁H₃₄N₄S=734.91) |
| 87 | m/z=758.25 (C₅₃H₃₄N₄S=758.93) | 88 | m/z=784.27 (C₅₅H₃₆N₄S=784.97) |
| 89 | m/z=810.28 (C₅₇H₃₈N₄S=811.00) | 90 | m/z=810.28 (C₅₇H₃₈N₄S=811.00) |
| 91 | m/z=758.25 (C₅₃H₃₄N₄S=758.93) | 92 | m/z=810.28 (C₅₇H₃₈N₄S=811.00) |
| 93 | m/z=708.23 (C₄₉H₃₂N₄S=708.87) | 94 | m/z=708.23 (C₄₉H₃₂N₄S=708.87) |
| 95 | m/z=810.28 (C₅₇H₃₈N₄S=811.00) | 96 | m/z=824.30 (C₅₈H₄₀N₄S=825.03) |
| 97 | m/z=748.27 (C₅₂H₃₆N₄S=748.93) | 98 | m/z=783.27 (C₅₆H₃₆N₃S=783.98) |
| 99 | m/z=810.28 (C₅₇H₃₈N₄S=811.00) | 100 | m/z=708.23 (C₄₉H₃₂N₄S=708.87) |
| 101 | m/z=758.25 (C₅₃H₃₄N₄S=758.93) | 102 | m/z=758.25 (C₅₃H₃₄N₄S=758.93) |
| 103 | m/z=810.28 (C₅₃H₃₈N₄S=811.00) | 104 | m/z=810.28 (C₅₇H₃₈N₄S=811.00) |
| 105 | m/z=810.28 (C₅₇H₃₈N₄S=811.00) | 106 | m/z=824.30 (C₅₈H₄₀N₄S=825.03) |
| 107 | m/z=810.28 (C₅₇H₃₈N₄S=811.00) | 108 | m/z=708.23 (C₄₉H₃₂N₄S=708.87) |
| 109 | m/z=708.23 (C₄₉H₃₂N₄S=708.87) | 110 | m/ z=632 .20 (C₄₃H₂₈N₄S=632.77) |
| 111 | m/z=734.25 (C₅₁H₃₄N₄S=734.91) | 112 | m/z=810.28 (C₅₇H₃₈N₄S=811.00) |
| 113 | m/z=708.23 (C₄₉H₃₂N₄S=708.87) | 114 | m/z=708.23 (C₄₉H₃₂N₄S=708.87) |
| 115 | m/z=748.27 (C₅₂H₃₆N₄S=748.93) | 116 | m/z=784.27 (C₅₅H₃₆N₄S=784.97) |
| 117 | m/z=784.27 (C₅₅H₃₆N₄S=784.97) | 118 | m/z=734.25 (C₅₁H₃₄N₄S=734.91) |
| 119 | m/z=758.25 (C₃₃H₃₄N₄S=758.93) | 120 | m/z=788.26 (C₅₄H₃₆N₄OS=788.95) |
| 121 | m/z=692.26 (C₄₉H₃₂N₄O=692.80) | 122 | m/z=691.26 (C₅₀H₃₃N₃O=691.82) |
| 123 | m/z=869.34 (C₆₄H₄₃N₃O=870.05) | 124 | m/z=870.34 (C₆₃H₄₂N₄O=871.03) |
| 125 | m/z=807.32 (C₅₉H₄₄N₃O=807.98) | 126 | m/z=844.32 (C₆₁H₄₀N₄O=845.00) |
| 127 | m/z=870.34 (C₆₃H₄₂N₄O=871.03) | 128 | m/z=747.23 (C₅₂H₃₃N₃OS=747.90) |
| 129 | m/z=718. 27 (C₅₁H₃₄N₄O=718.84) | 130 | m/z=781.27 (C₅₆H₃₅N₃O₂=781.90) |
| 131 | m/z=817.31 (C₆₀H₃₉N₃O=817.97) | 132 | m/z=691.26 (C₅₀H₃₃N₃O=691.82) |
| 133 | m/z=767.29 (C₅₆H₃₇N₃O=767.91) | 134 | m/z=691.26 (C₅₀H₃₃N₃O=691.82) |
| 135 | m/z=870.34 (C₆₃H₄₂N₄O=871.03) | 136 | m/z=757.31 (C₅₅H₃₉N₃O=757.92) |
| 137 | m/z=848.32 (C₆₀H₄₀N₄O₂=848.99) | 138 | m/z=794.30 (C₅₇H₃₈N₄O=794.94) |
| 139 | m/z=818.30 (C₅₉H₃₈N₄O=818.96) | 140 | m/z=843.32 (C₆₂H₄₄N₃O=844.01) |
| 141 | m/z=641.25 (C₄₆H₃₄N₃O=641.76) | 142 | m/z=718.27 (C₅₁H₃₄N₄O=718.84) |
| 143 | m/z=793.31 (C₅₈H₃₉N₃O=793.95) | 144 | m/z=794.30 (C₅₇H₃₈N₄O=794.94) |
| 145 | m/z=787.27 (C₅₅H₃₇N₃OS=787.97) | 146 | m/z=717.28 (C₅₂H₃₅N₃O=717.85) |
| 147 | m/z=767.29 (C₅₈H₃₇N₃O=767.91) | 148 | m/z=781.27 (C₅₆H₃₅N₃O₂=781.90) |
| 149 | m/z=731.29 (C₅₃H₃₇N₃O=731.88) | 150 | m/z=767.29 (C₅₆H₃₇N₃O=767.91) |
| 151 | m/z=818.30 (C₅₉H₃₈N₄O=818.96) | 152 | m/z=823.27 (C₅₈H₃₇N₃OS=824.00) |
| 153 | m/z=692.26 (C₄₉H₃₂N₄O=692.80) | 154 | m/z=767.29 (C₅₆H₃₇N₃O=767.91) |
| 155 | m/z=843.32 (C₆₂H₄₄N₃O=844.01) | 156 | m/z=834.34 (C₆₀H₄₂N₄O=835.00) |
| 157 | m/z=757.31 (C₅₅H₃₉N₃O=757.92) | 158 | m/z=717.28 (C₅₂H₃₅N₃O=717.85) |
| 159 | m/z=768.29 (C₅₅H₃₆N₄O=768.90) | 160 | m/z=823.27 (C₅₈H₃₇N₃OS=824.00) |
| 161 | m/z=657.22 (C₄₆H₃₁N₃S=657.82) | 162 | m/z=734.25 (C₅₁H₃₄N₄S=734.91) |
| 163 | m/z=823.27 (C₅₈H₃₇N₃OS=824.00) | 164 | m/z=809.29 (C₅₈H₃₉N₃S=810.02) |
| 165 | m/z=774.28 (C₅₄H₃₈N₄S=774.97) | 166 | m/z=783.27 (C₅₆H₃₇N₃S=783.98) |
| 167 | m/z=810.28 (C₅₇H₃₈N₄S=811.00) | 168 | m/z=733.26 (C₅₂H₃₅N₃S=733.92) |
| 169 | m/z=849.32 (C₆₁H₄₃N₃S=850.08) | 170 | m/z=784.27 (C₅₅H₃₆N₄S=784.97) |
| 171 | m/z=834.28 (C₅₉H₃₈N₄S=835.02) | 172 | m/z=809.29 (C₅₈H₃₉N₃S=810.02) |
| 173 | m/z=708.23 (C₄₉H₃₂N₄S=708.87) | 174 | m/z=784.27 (C₅₅H₃₆N₄S=784.97) |
| 175 | m/z=783.27 (C₅₆H₃₇N₃S=783.98) | 176 | m/z=849.32 (C₆₁H₄₃N₃S=850.08) |
| 177 | m/z=863.30 (C₆₁H₄₁N₃OS=864.06) | 178 | m/z=784.27 (C₅₅H₃₆N₄S=784.97) |
| 179 | m/z=784.27 (C₅₅H₃₆N₄S=784.97) | 180 | m/z=733.26 (C₅₂H₃₅N₃S=733.92) |
| 181 | m/z=727. 85 (C₅₃H₃₃N₃O=727.26) | 183 | m/z=650.76 (C₄₈H₃₀N₂O=650.24) |
| 185 | m/z=694.80 (C₄₈H₂₆N₂O₂S=694.17) | 187 | m/z=680.81 (C₄₈H₂₈N₂OS=680.19) |
| 189 | m/z=701.81 (C₅₁H₃₁NO₃=701.25) | 191 | m/z=650.76 (C₄₈H₃₀N₂O=650.24) |
| 193 | m/z=651. 75 (C₄₇H₂₉N₃O=651.23) | 195 | m/z=650.76 (C₄₈H₃₀N₂O=650.24) |
| 197 | m/z=651. 75 (C₄₇H₂₉N₃O=651.23) | 199 | m/z=651. 75 (C₄₇H₂₉N₃O=651.23) |

**[Table 3]**

| Compound | ¹H NMR (CDCl₃, 200 Mz) |
|---|---|
| 1 | δ=8.28 (2H, d), 7.75 (1H, d), 7.64 (1H, d), 7.62 (1H, d), 7.51-7.41 (8H, m), 7.20 (4H, t), 6.81 (2H, t), 6.63 (4H, d), 6.33 (1H, d) |
| 3 | δ=8.28 (4H, d), 7.75 (1H, d), 7.64 (1H, d), 7.62 (1H, d), 7.54-7.41 (22H, m), 6.69 (4H, d), 6.33 (1H, d), |
| 6 | δ=8.28 (4H, d), 7.75 (1H, d), 7.64 (1H, d), 7.62 (1H, d), 7.54-7.41 (21H, m), 6.89-88 (2H, m), 6.69 (2H, d), 6.59 (1H, d), 6.33 (1H, d) . |
| 10 | δ=8.28 (4H, d), 8.02-7.95 (3H, m), 7.75 (2H, d), 7.62-7.41 (14H, m), 7.20 (2H, t), 6.98 (1H, d), 6.89-6.81 (3H, m), 6.63-6.59 (3H, m) . |
| 15 | δ=8.55 (1H, m), 8.28 (2H, d), 8.08-8.04 (2H, m), 7.95 (1H, d), 7.75 (1H, d), 7.62-7.41 (24H, m), 6.69 (4H, d), 6.33 (1H, d). |
| 19 | δ=8.28 (4H, d), 7.78-7.74 (5H, m), 7.64-7.62 (2H, m), 7.52-7.36 (17H, m), 6.89-6.88 (2H, m), 6.59 (1H, d), 6.33 (1H, d). |
| 22 | δ=8.28 (2H, d), 7.85 (2H, d), 7.75 (2H, d), 7.64-7.62 (2H, m), 7.54-7.41 (18H, m), 7.25 (2H, d), 7.20 (2H, d), 6.81 (1H, t), 6.69 (2H, d), 6.63 (2H, d), 6.33 (1H, d). |
| 27 | δ=8.28-8.24 (3H, m), 7.75-7.41 (26H, m), 7.20 (2H, t), 7.06 (2H, s), 6.85-6.81 (3H, m), 6.63 (2H, d), 6.33 (1H, d). |
| 29 | δ=9.09 (1H, s), 8.49 (1H, d), 8.28 (2H, d), 8.00-7.92 (4H, m), 7.75 (2H, d), 7.64-7.41 (9H, m), 7.20 (4H, t), 6.89-6.81 (4H, m), 6.63-6.59 (5H, m). |
| 32 | δ=8.28-8.24 (3H, m), 7.75-7.70 (2H, m), 7.62-7.41 (27H, m), 6.89-6.88 (2H, m), 6.69 (2H, d), 6.59 (1H, d), 6.33 (1H, d). |
| 35 | δ=8.28 (2H, d), 7.85-7.75 (5H, m), 7.64-7.62 (2H, m), 7.54-7.41 (24H, m), 6.69 (4H, d), 6.33 (1H, d). |
| 39 | δ=8.55 (1H, m), 8.28 (2H, d), 7.95-7.74 (6H, m), 7.64-7.36 (19H, m), 6.89-6.88 (2H, m), 6.59 (1H, d), 6.33 (1H, d) . |
| 41 | δ=8.55 (1H, m), 8.28 (2H, d), 8.08-8.04 (2H, m), 7.95 (1H, d), 7.75 (1H, d), 7.62-7.41 (10H, m), 7.20 (4H, t), 6.81 (2H, t), 6.63 (4H, d), 6.33 (1H, d). |
| 47 | δ=8.28-8.24 (3H, m), 7.75-7.41 (26H, m), 7.20 (2H, t), 7.06 (2H, s), 6.85-6.81 (3H, m), 6.63 (2H, d), 6.33 (1H, d). |
| 52 | δ=8.28-8.24 (3H, m), 7.75-7.41 (29H, m), 6.89-6.88 (2H, m), 6.69 (2H, d), 6.59 (1H, d), 6.33 (1H, d). |
| 55 | δ=8.28 (2H, d), 7.85-7.75 (5H, m), 7.64-7.62 (2H, m), 7.54-7.41 (24H, m), 6.69 (4H, d), 6.33 (1H, d). |
| 60 | δ=9.09 (1H, s), 8.49 (1H, d), 8.28 (2H, d), 8.00-7.92 (4H, m), 7.75 (2H, d), 7.64-7.41 (14H, m), 7.20-7.16 (3H, m), 7.08 (2H, d), 6.69-6.63 (5H, m). |
| 62 | δ=8.28 (4H, d), 7.94 (1H, d), 7.82-7.80 (2H, m), 7.56-7.41 (14H, m), 7.20 (2H, t), 7.06 (1H, s), 6.88-6.81 (2H, m), 6.69 (2H, d), 6.63 (1H, d). |
| 72 | δ=8.28 (4H, d), 7.94 (1H, d), 7.82-7.80 (2H, m), 7.56-7.41 (20H, m), 7.06 (1H, s), 6.89-6.88 (2H, m), 6.69 (2H, d), 6.59 (1H, d). |
| 74 | δ=9.09 (1H, s), 8.49 (1H, d), 8.28 (2H, d), 8.00-7.92 (4H, m), 7.82-7.80 (2H, m), 7.59-7.41 (13H, m), 7.20 (2H, t), 7.06 (1H, s), 6.88-6.63 (6H, m). |
| 80 | δ=8.28 (4H, d), 8.11-8.05 (3H, m), 7.94 (1H, d), 7.82 (1H, d), 7.56-7.41 (13H, m), 7.20-7.08 (5H, m), 6.87-6.81 (2H, m), 6.69 (3H, d), 6.63 (2H, d). |
| 86 | δ=8.28 (2H, m), 7.94 (1H, d), 7.82-7.80 (2H, m), 7.57-7.41 (17H, m), 7.20 (2H, t), 7.06 (1H, s), 6.89-6.81 (4H, m), 6.36-6.59 (3H, m) |
| 90 | δ=8.28 (2H, d), 7.94 (1H, d), 7.85-7.79 (6H, m), 7.56-7.41 (16H, m), 7.25 (4H, s), 7.20 (1H, t), 7.06 (1H, s), 6.88-6.81 (2H, m), 6.69 (2H, d), 6.63 (2H, d). |
| 95 | δ=8.28 (2H, d), 7.94 (1H, d), 7.85-7.79 (6H, m), 7.56-7.41 (23H, m), 7.06 (1H, s), 6.88 (2H, d), 6.69 (4H, d). |
| 99 | δ=8.28 (2H, d), 8.24 (1H, d), 7.94 (1H, d), 7.82-7.80 (2H, m), 7.70 (1H, s), 7.57-7.41 (21H, m), 7.20 (2H, d), 7.06 (2H, s), 6.88-6.81 (4H, m), 6.63 (2H, d). |
| 103 | δ=8.28 (2H, d), 8.24 (1H, d), 7.94 (1H, d), 7.82-7.80 (2H, m), 7.70 (1H, s), 7.57-7.41 (22H, m), 7.06 (2H, s), 6.89-6.88 (3H, m), 6.69 (2H, d), 6.59 (1H, m). |
| 105 | δ=8.28 (2H, d), 7.94 (1H, d), 7.85-7.79 (6H, m), 7.56-7.41 (23H, m), 7.06 (1H, s), 6.88 (1H, d), 6.69 (4H, d). |
| 111 | δ=8.28 (2H, d), 7.94 (1H, d), 7.85-7.79 (6H, m), 7.56-7.41 (23H, m), 7.06 (1H, s), 6.88 (1H, d), 6.69 (4H, d). |
| 118 | δ=8.28 (2H, d), 7.94 (1H, d), 7.85-7.79 (6H, m), 7.56-7.41 (23H, m), 7.06 (1H, s), 6.88 (1H, d), 6.69 (4H, d). |
| 126 | δ=8.55 (1H, d), 8.28-8.24 (3H, m), 8.08-8.04 (2H, m), 7.75 (1H, d), 7.70 (1H, s), 7.64-7.41 (25H, m), 6.89 (1H, s), 6.88 (1H, d), 6.69 (2H, d), 6.59 (1H, d), 6.33 (1H, d). |
| 133 | δ=8.30 (2H, d), 8.03-8.02 (2H, m), 7.79 (2H, d), 7.85 (2H, d), 7.62-7.38 (18H, m), 7.20 (2H, t), 6.98 (1H, d), 6.81 (1H, t), 6.63 (2H, d), 6.33 (1H, d). |
| 144 | δ=8.28 (4H, d), 7.85 (2H, d), 7.75 (1H, d), 7.64-7.62 (2H, m), 7.54-7.41 (17H, m), 7.25 (4H, d), 7.20 (2H, d), 6.81 (1H, t), 6.69 (2H, d), 6.63 (2H, d), 6.33 (1H, d). |
| 151 | δ=8.28 (4H, d), 8.02-7.49 (11H, m), 7.57-7.36 (19H, m), 6.98 (1H, d), 6.89-6.88 (2H, m), 6.59 (1H, d). |
| 160 | δ=8.05 (1H, d), 7.98 (1H, d), 7.95 (1H, d), 7.85 (2H, d), 7.79 (2H, d), 7.75 (2H, m), 7.64-7.41 (14H, m), 7.25-08 (7H, m), 6.87-6.81 (2H, m), 6.69 (2H, d), 6.63 (2H, d). |
| 161 | δ=8.24-8.23 (2H, m), 7.94 (1H, d), 7.82-7.79 (6H, m), 7.70 (1H, s), 7.57-7.41 (9H, m), 7.20 (4H, t), 7.06 (1H, s), 6.88 (1H, d), 6.81 (2H, m), 6.63 (4H, d). |
| 171 | δ=8.28 (4H, d), 8.11-8.02 (5H, m), 7.88-7.74 (4H, m), 7.94-7.74 (8H, m), 7.57-7.38 (17H, m), 6.98 (1H, d), 6.89-88 (2H, m), 6.59 (1H, d). |
| 179 | δ=8.28 (4H, d), 7.94-7.74 (9H, m), 7.52-7.36 (18H, m), 7.06 (1H, s), 6.89-6.88 (3H, m), 6.59 (1H, d). |

### [Preparation Example 2] Preparation of Compound 183

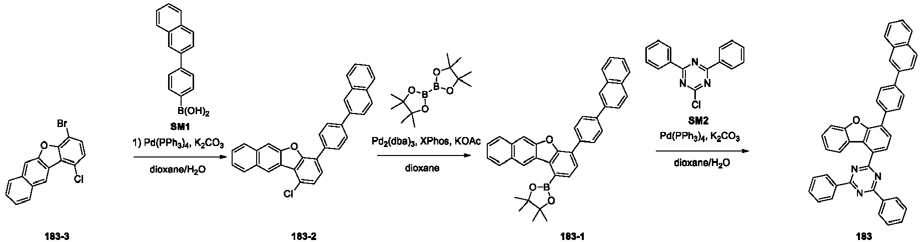

### Synthesis of Compound 183-2

Compound 183-3 (5 g, 13.5 mmol), Compound SM1 (4.6 g, 16.2 mmol), Pd(PPh₃)₄ (tetrakis(triphenylphosphine)palladium(0)) (0.8 g, 0.67 mmol) and K₂CO₃ (3.7 g, 27 mmol) were introduced to a 250 mL round bottom flask, and after creating a nitrogen atmosphere, 1,4-dioxane (80 mL) and H₂O (20 mL) were introduced thereto, and the mixture was stirred for 12 hours at 120°C. After the reaction was terminated, the reaction temperature was lowered to room temperature, and the result was washed with water and extracted with methylene chloride. The extracted organic solution was dried with Mg₂SO₄, and then concentrated. The result was silica gel columned and recrystallized to obtain white solid Compound 183-2 (2.9 g, 6.5 mmol, yield 48%).

### Synthesis of Compound 183-1

Compound 183-2 (2.9 g, 6.5 mmol), 4,4,4',4',5,5,5',5'-octamethyl-2,2'-bi(1,3,2-dioxaborolane) (2.5 g, 9.8 mmol), Pd₂(dba)₃ (tris(dibenzylideneacetone)dipalladium(0)) (0.3 g, 0.3 mmol), XPhos (2-dicyclohexylphosphino-2',4',6'-triisopropylbiphenyl) (1.2 g, 2.6 mmol) and KOAc (1.3 g, 13 mmol) were introduced to a 100 mL round bottom flask, and after creating a nitrogen atmosphere, 1,4-dioxane (50 mL) was introduced thereto, and the mixture was stirred for 3 hours at 110°C. After the reaction was terminated, the reaction temperature was lowered to room temperature, and the result was washed with water and extracted with methylene chloride. The extracted organic solution was dried with Mg₂SO₄, and then concentrated. The result was silica gel columned and recrystallized to obtain white solid Compound 183-1 (3.0 g, 5.54 mmol, yield 85%) .

### Synthesis of Compound 183

Compound 183-1 (3.0 g, 5.54 mmol), Compound SM2 (1.8 g, 6.6 mmol), Pd(PPh₃)₄ (0.3 g, 0.27 mmol) and K₂CO₃ (1.5 g, 11 mmol) were introduced to a 100 mL round bottom flask, and after creating a nitrogen atmosphere, 1,4-dioxane (40 mL) and H₂O (10 mL) were introduced thereto, and the mixture was stirred for 4 hours at 120°C. After the reaction was terminated, the reaction temperature was lowered to room temperature, and the result was washed with water and extracted with methylene chloride. The extracted organic solution was dried with Mg₂SO₄, and then concentrated. The result was silica gel columned and recrystallized to obtain yellow solid Compound 183 (3.1 g, 4.7 mmol, yield 85%).

Target compounds were synthesized in the same manner as in Preparation Example 2 except that Intermediate A of the following Table 4 was used instead of SM1, and Intermediate B of the following Table 4 was used instead of SM2.

**[Table 4]**

| Compound No. | Intermediate A | Intermediate B | Target Compound | Yield |
|---|---|---|---|---|
| 182 | 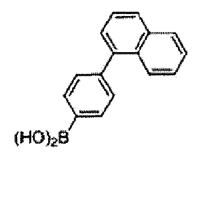 | 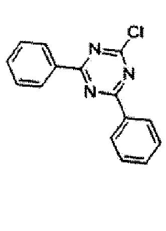 | 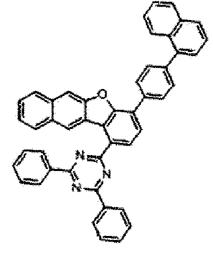 | 50% |
| 183 | 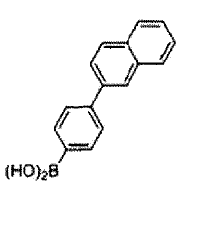 | 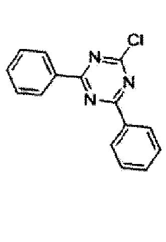 | 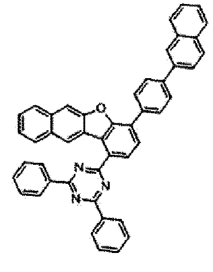 | 66% |
| 186 | 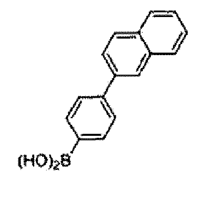 | 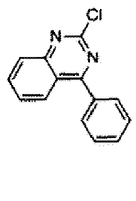 | 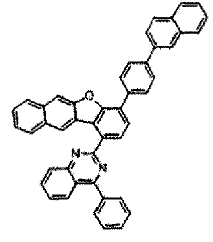 | 12% |
| 187 | 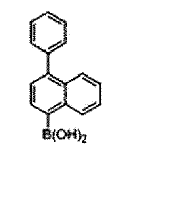 | 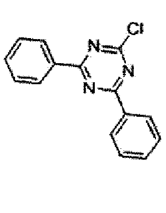 | 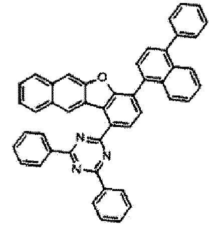 | 53% |

### <Experimental Example 1>

### 1) Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. Specifically, a light emitting layer having a thickness of 500 Å was deposited by using compounds described in Examples 1 to 5 and Comparative Examples 1 to 4 of the following Table 5 as a red host of the light emitting layer, and doping (piq)₂(Ir) (acac), a red phosphorescent dopant, to the red host by 3 wt%. After that, bathocuproine (hereinafter, BCP) was deposited to 60 Å as a hole blocking layer, and Alq₃ was deposited to 200 Å thereon as an electron transfer layer.

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic light emitting device was manufactured.

In addition, organic light emitting devices were manufactured in the same manner as the organic light emitting devices of Examples 1 to 5 and Comparative Examples 1 to 4 except that, in the process of depositing the light emitting layer, a light emitting layer having a thickness of 500 Å was deposited by using compounds described in Examples 6 to 8 of the following Table 5 as a green host, and doping Ir(ppy)₃, a green phosphorescent dopant, to the green host by 7 wt% based on a total weight of the light emitting layer.

Meanwhile, all the organic compounds required to manufacture the OLED were vacuum sublimation purified under 10⁻⁸ torr to 10⁻⁶ torr for each material to be used in the OLED manufacture.

Specifically, Compounds A to D used in Comparative Examples 1 to 4 are as follows.

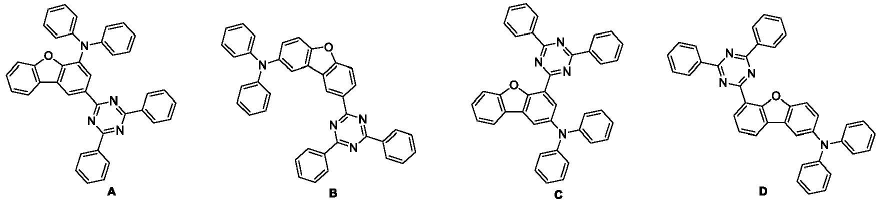

### 2) Driving Voltage and Light Emission Efficiency of Organic Light Emitting Device

For each of the organic light emitting devices of Examples 1 to 8 and Comparatives Example 1 to 4 manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc.. The T₉₀ means a lifetime (unit: h, time), a time taken to become 90% with respect to initial luminance.

Measured properties of the organic light emitting devices are as shown in the following Table 5.

**[Table 5]**

| | Comp ound | Threshold Voltage (Vₒₙ) | Driving Voltage (Vₒₚ) | Efficie ncy (cd/A) | Color Coordinate (x, y) | Life time (T₉₀) |
|---|---|---|---|---|---|---|
| Comparative Example 1 | A | 2.10 | 6.32 | 10.2 | (0.677, 0.323) | 3 |
| Comparative Example 2 | B | 2.15 | 6.01 | 8.9 | (0.676, 0.324) | N/D |
| Comparative Example 3 | C | 2.21 | 6.21 | 9.7 | (0.673, 0.319) | 2 |
| Comparative Example 4 | D | 2.23 | 6.10 | 9.1 | (0.673, 0.321) | N/D |
| Example 1 | 6 | 2.30 | 5.12 | 25.3 | (0.673, 0.319) | 59 |
| Example 2 | 46 | 2.42 | 5.03 | 25.4 | (0.675, 0.325) | 62 |
| Example 3 | 72 | 2.28 | 4.98 | 26.2 | (0.684, 0.316) | 44 |
| Example 4 | 101 | 2.21 | 4.87 | 23.9 | (0.673, 0.327) | 63 |
| Example 5 | 168 | 2.53 | 4.92 | 22.7 | (0.684, 0.316) | 51 |
| Example 6 | 82 | 2.63 | 3.94 | 45.4 | (0.283, 0.687) | 30 |
| Example 7 | 142 | 2.70 | 3.88 | 50.2 | (0.289, 0.676) | 34 |
| Example 8 | 170 | 2.61 | 3.80 | 46.4 | (0.274, 0.668) | 29 |

When using a compound with a high molecular weight as a material of a light emitting layer, the deposition process is difficult to proceed since vaporization is difficult due to high intermolecular bonding strength, and using a compound with a low molecular weight may cause difficulties in solvent selection, however, the heterocyclic compound represented by Chemical Formula 1 according to the present application has a molecular weight suitable to be used in a light emitting layer of an organic light emitting device while having high thermal stability. This means that the compound represented by Chemical Formula 1 according to the present application is a material to readily form a light emitting layer of an organic light emitting device. In addition, the band gap that the compound represented by Chemical Formula 1 has prevents losses of electrons and holes of the light emitting layer helping with effective formation of recombination zone.

In other words, it may be identified that, in a compound having a substituted arylamine group attached at a proper position like the heterocyclic compound represented by Chemical Formula 1 according to the present application, strong hole transfer (HT) properties of the arylamine group substituting at the position resolves a hole trap phenomenon occurring in the dopant. In addition, the threshold voltage may be adjusted depending on the electron transfer (ET) group.

Specifically, it was identified that the heterocyclic compound represented by Chemical Formula 1 of the present application had electron clouds of HOMO and LUMO being separated without being spread. On the other hand, it was identified that Comparative Compounds A to D had electron clouds of HOMO and LUMO being spread. As electron clouds are more separated without being spread, a hole trap phenomenon occurring in the dopant is more readily resolved.

In addition, it was identified that, when calculating an oscillator strength value as in the following Table 6, the value was significantly low in Comparative Compounds A to D compared to in the heterocyclic compound represented by Chemical Formula 1 of the present application. This means that the heterocyclic compound represented by Chemical Formula 1 of the present application has more superior fluorescent material absorbance. In other words, it was identified that, in the organic light emitting device using the heterocyclic compound represented by Chemical Formula 1 of the present application, the compound was a material more superior to be used in the light emitting layer.

Lastly, it was also identified that the heterocyclic compound represented by Chemical Formula 1 of the present application had a higher photoluminescence quantum yield (PLQY) value compared to Comparative Compounds A to D. As a result, it was identified that the organic light emitting device using the heterocyclic compound represented by Chemical Formula 1 of the present application had a more superior device lifetime.

Such a difference is considered to be due to the fact that a substituted arylamine group substitutes at a proper position in the heterocyclic compound represented by Chemical Formula 1 according to the present application.

**[Table 6]**

| **name** | Structural formula | HOMO | | Eg |
|---|---|---|---|---|
| 14 | 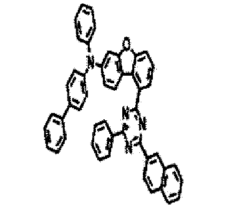 | -5.26 | 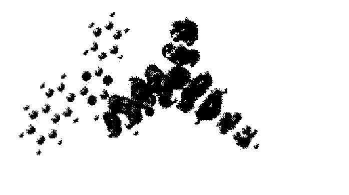 | 2.94 |
| 28 | 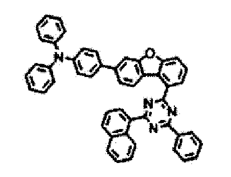 | -5.27 | 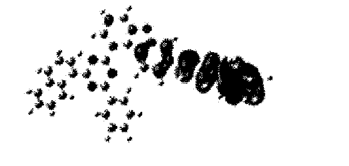 | 2.99 |
| A | 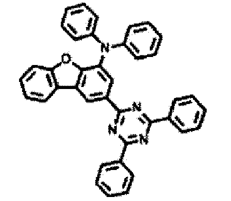 | -5.41 | 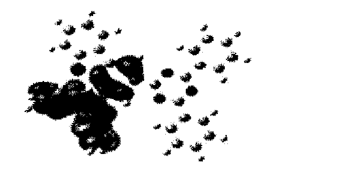 | 3.23 |
| B | 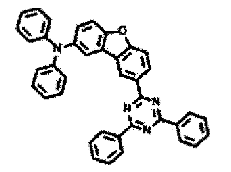 | -533 | 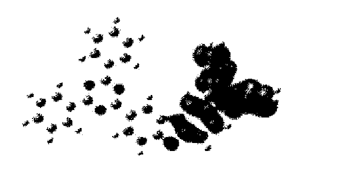 | 3.13 |
| C | 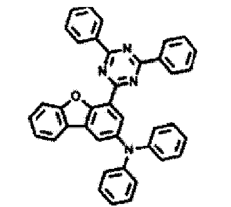 | -5.33 | 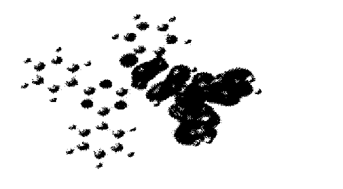 | 3.07 |
| D | 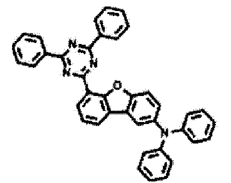 | -5.32 | 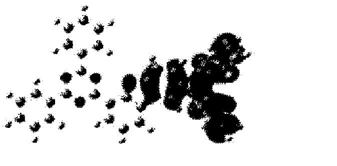 | 3.05 |
| 14 | 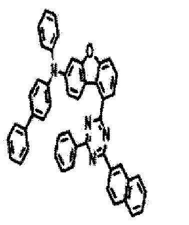 | -2.31 | 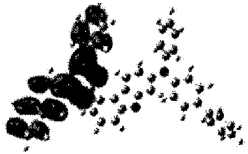 | 2.58 |
| 28 | 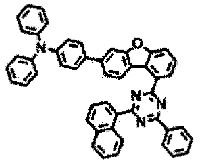 | -2.28 | 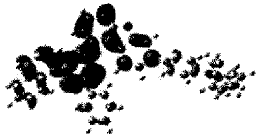 | 2.65 |
| A | 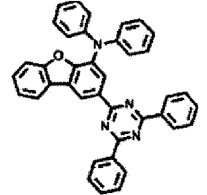 | -2.18 | 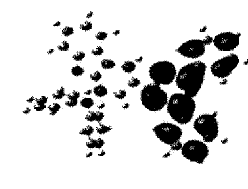 | 2.79 |
| B | 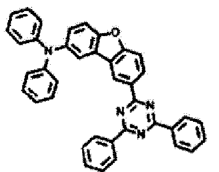 | -2.20 | 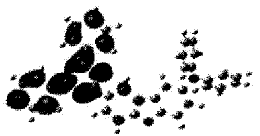 | 2.80 |
| C | 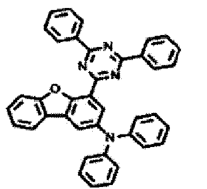 | -2.26 | 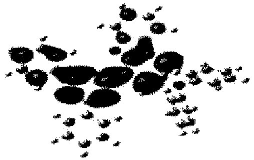 | 2.53 |
| D | 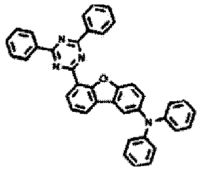 | -2.27 | 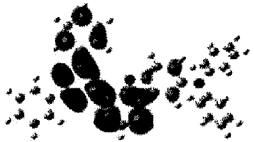 | 2.64 - |

| **name** | Structural formula | T₁ | Dipole moment | Oscillator Strength |
|---|---|---|---|---|
| 14 | 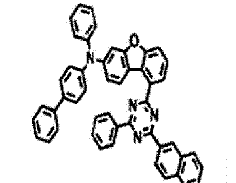 | 2.33 | 1.11 | 0.0025 |
| 28 | 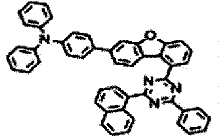 | 2.42 | 1.22 | 0.2559 |
| A | 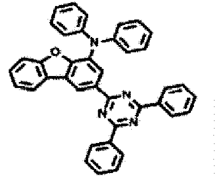 | 2.53 | 0.53 | 0.0183 |
| B | 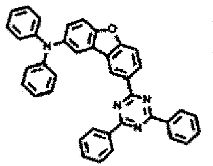 | 2.71 | 0.69 | 0.0007 |
| C | 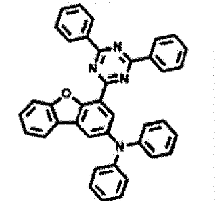 | 2.25 | 0.72 | 0.0353 |
| D | 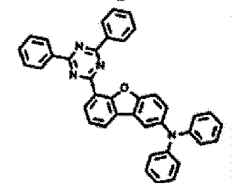 | 2.50 | 0.63 | 0.0097 |

Accordingly, as seen from the evaluation on device of Table 5, the heterocyclic compound represented by Chemical Formula 1 according to the present application exhibited improved performance compared to the comparative examples. In other words, as seen from the evaluation on device of Table 5, using the heterocyclic compound represented by Chemical Formula 1 according to the present application in the light emitting layer of the organic light emitting device resulted in more superior effects in the properties of lifetime, efficiency and driving voltage compared to the cases of not using the heterocyclic compound.

### <Experimental Example 2>

### 1) Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a first hole injection layer 2-TNATA (4,4',4"-tris[2-naphthyl(phenyl)amino]triphenylamine) and a first hole transfer layer NPB (N,N'-di(1-naphthyl)-N,N'-diphenyl-(1,1'-biphenyl)-4,4'-diamine) were formed.

A first light emitting layer was thermal vacuum deposited thereon as follows. Specifically, a first light emitting layer having a thickness of 400 Å was deposited by mixing the heterocyclic compound represented by Chemical Formula 1 of the present application described in the following Table 7, which is a first host, and the heterocyclic compound represented by Chemical Formula 2 of the present application described in the following Table 7, which is a second host, in a ratio described in the following Table 7 as a red host of the first light emitting layer, and doping (piq)₂(Ir)(acac), a red phosphorescent dopant, to the red host by 2 wt% based on a total weight of the first light emitting layer.

After that, Alq₃ was deposited to 120 Å as a first electron transfer layer, Bphen was deposited to 120 Å thereon as an N-type charge generation layer, and MoO₃ was deposited to 100 Å thereon as a P-type charge generation layer. In addition, on the P-type charge generation layer, a second hole transfer layer NPB (N,N'-di(1-naphthyl)-N,N'-diphenyl-(1,1'-biphenyl)-4,4'-diamine) was formed.

A second light emitting layer was thermal vacuum deposited thereon as follows. Specifically, a second light emitting layer having a thickness of 400 Å was deposited by mixing the heterocyclic compound represented by Chemical Formula 1 of the present application described in the following Table 7, which is a first host, and the heterocyclic compound represented by Chemical Formula 2 of the present application described in the following Table 7, which is a second host, in a ratio described in the following Table 7 as a red host of the second light emitting layer, and doping (piq)₂(Ir) (acac), a red phosphorescent dopant, to the red host by 2 wt% based on a total weight of the second light emitting layer.

After that, Alq₃ was deposited to 300 Å as a second electron transfer layer. Lastly, an electron injection layer was formed on the second electron transfer layer by depositing lithium fluoride (LiF) to a thickness of 20 Å, and then a cathode was formed on the electron injection layer by depositing an aluminum (Al) cathode to a thickness of 1,200 Å, and as a result, an organic electroluminescent device of each of Comparative Example 5 and Examples 9 to 30 was manufactured.

Meanwhile, all the organic compounds required to manufacture the OLED were vacuum sublimation purified under 10⁻⁸ torr to 10⁻⁶ torr for each material to be used in the OLED manufacture.

In addition, the heterocyclic compound represented by Chemical Formula 1 according to the present application corresponds to a P-type host compound, and the heterocyclic compound represented by Chemical Formula 2 according to the present application corresponds to an N-type host compound. Accordingly, N:P of the following Table 7 means a weight ratio of the N-type host compound (compound represented by Chemical Formula 2) and the P-type host compound (compound represented by Chemical Formula 1). More specifically, it means a weight ratio of the first host and the second host.

### 2) Driving Voltage and Light Emission Efficiency of Organic Light Emitting Device

For each of the organic light emitting devices of Comparative Example 5 and Examples 9 to 30 manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc..

Properties of the organic electroluminescent devices of the present disclosure are as shown in the following Table 7.

**[Table 7]**

| | Compound (First Host:Seco nd Host) | Ratio (N:P) | Threshold Voltage (V_{cn}) | Driving Voltage (V_{cp}) | Effici ency (cd/A) | Color Coordinate (x, y) | Lifet ime (T₉₀) |
|---|---|---|---|---|---|---|---|
| Comparative Example 5 | A:211 | 1:1 | 4.10 | 6.21 | 9.7 | 0.673, 0.319 | 10 |
| Example 9 | 6:242 | 1:2 | 4.49 | 5.18 | 79.87 | 0.676, 0.324 | 702 |
| Example 10 | | 1:1 | 4.66 | 5.20 | 86.51 | 0.679, 0.321 | 670 |
| Example 11 | | 2:1 | 4.68 | 5.31 | 90.36 | 0.674, 0.326 | 615 |
| Example 12 | 44:227 | 1:1 | 4.07 | 5.15 | 90.50 | 0.673, 0.327 | 681 |
| Example 13 | 50 :227 | 1:1 | 4.52 | 5.63 | 90.82 | 0.674, 0.326 | 690 |
| Example 14 | 88:193 | 1:1 | 4.66 | 6.01 | 93.44 | 0.681, 0.319 | 481 |
| Example 15 | 100:238 | 1:1 | 4.32 | 6.27 | 91.82 | 0.673, 0.327 | 492 |
| Example 16 | 100:224 | 1:1 | 4.38 | 5.98 | 91.53 | 0.682, 0.318 | 598 |
| Example 17 | 107:232 | 1:1 | 4.27 | 6.27 | 87.39 | 0.673, 0.327 | 483 |
| Example 18 | 115:259 | 1:1 | 4.25 | 5.98 | 89.13 | 0.682, 0.318 | 512 |
| Example 19 | 122:219 | 1:1 | 4.31 | 5.82 | 90.02 | 0.682, 0.318 | 498 |
| Example 20 | 128:253 | 1:1 | 4.48 | 5.69 | 88.34 | 0.673, 0.327 | 502 |
| Example 21 | 138:259 | 1:2 | 4.49 | 5.18 | 95.71 | 0.682, 0.318 | 710 |
| Example 22 | | 1:1 | 4.66 | 5.20 | 101.23 | 0.682, 0.318 | 692 |
| Example 23 | | 2:1 | 4.68 | 5.33 | 102.12 | 0.673, 0.327 | 621 |
| Example 24 | 142:242 | 1:2 | 4.32 | 5.15 | 103.89 | 0.676, 0.324 | 780 |
| Example 25 | 147:256 | 1:1 | 4.67 | 5.57 | 91.20 | 0.677, 0.323 | 755 |
| Example 26 | 154:243 | 2:1 | 4.92 | 5.67 | 98.20 | 0.678, 0.322 | 598 |
| Example 27 | 160:229 | 3:1 | 4.82 | 5.61 | 98.82 | 0.676, 0.324 | 539 |
| Example 28 | 162:186 | 1:2 | 4.52 | 5.72 | 97.80 | 0.684, 0.316 | 702 |
| Example 29 | 174:197 | 1:1 | 4.58 | 5.80 | 95.28 | 0.685, 0.315 | 679 |
| Example 30 | 175:259 | 1:2 | 4.62 | 5.18 | 89.25 | 0.676, 0.324 | 509 |

As seen from the evaluation on device of Table 7, it was identified that effects of more superior driving, efficiency and lifetime were obtained in the organic light emitting device when mixing and depositing the heterocyclic compound represented by Chemical Formula 1 and the heterocyclic compound represented by Chemical Formula 2 according to the present application as the organic material layer of the organic light emitting device.

This result may be considered as a result of improving a phenomenon of reducing a lifetime of the compound comprising an arylamine group. It may be expected that an exciplex phenomenon occurred when comprising the heterocyclic compound represented by Chemical Formula 1 according to the present application corresponding to a P-type host compound having strong hole transfer properties and the heterocyclic compound represented by Chemical Formula 2 according to the present application corresponding to an N-type host compound at the same time.

The exciplex phenomenon is a phenomenon of releasing energy having sizes of a donor (p-host) HOMO level and an acceptor (n-host) LUMO level due to electron exchanges between two molecules of an N-type host compound and a P-type host compound. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime.

### <Reference Numeral>

- 100:: Substrate
- 200:: Anode
- 300:: Organic Material Layer
- 301:: Hole Injection Layer
- 302:: Hole Transfer Layer
- 303:: Light Emitting Layer
- 304:: Hole Blocking Layer
- 305:: Electron Transfer Layer
- 306:: Electron Injection Layer
- 400:: Cathode

## Claims

1. A heterocyclic compound represented by the following Chemical Formula 1: 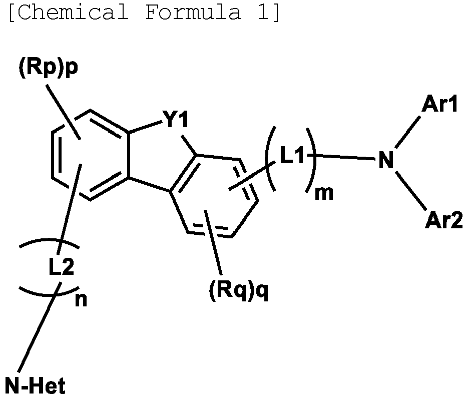
wherein, in Chemical Formula 1,
Y1 is O; or S;
L1 and L2 are the same as or different from each other, and each independently a direct bond; a substituted or unsubstituted arylene group having 6 to 60 carbon atoms; or a substituted or unsubstituted heteroarylene group having 2 to 60 carbon atoms, m and n are each an integer of 0 to 3, and when m and n are each 2 or greater, substituents in the parentheses are the same as or different from each other;
N-Het is a monocyclic or polycyclic heterocyclic group substituted or unsubstituted and comprising one or more Ns;
Ar1 and Ar2 are the same as or different from each other, and each independently a substituted or unsubstituted aryl group having 6 to 60 carbon atoms; or a substituted or unsubstituted heteroaryl group having 2 to 60 carbon atoms; and
Rp and Rq are the same as or different from each other, and each independently hydrogen; deuterium; a halogen group; or a substituted or unsubstituted alkyl group having 1 to 60 carbon atoms, p and q are each an integer of 0 to 3, and when p and q are each 2 or greater, substituents in the parentheses are the same as or different from each other.

2. The heterocyclic compound of Claim 1, wherein the substituted or unsubstituted means being substituted with one or more substituents selected from the group consisting of deuterium; a cyano group; a halogen group; linear or branched alkyl having 1 to 60 carbon atoms; linear or branched alkenyl having 2 to 60 carbon atoms; linear or branched alkynyl having 2 to 60 carbon atoms; monocyclic or polycyclic cycloalkyl having 3 to 60 carbon atoms; monocyclic or polycyclic heterocycloalkyl having 2 to 60 carbon atoms; monocyclic or polycyclic aryl having 6 to 60 carbon atoms; monocyclic or polycyclic heteroaryl having 2 to 60 carbon atoms; -SiRR'R"; - P(=O)RR'; alkylamine having 1 to 20 carbon atoms; monocyclic or polycyclic arylamine having 6 to 60 carbon atoms; and monocyclic or polycyclic heteroarylamine having 2 to 60 carbon atoms, or being unsubstituted, or being substituted with a substituent linking two or more substituents selected from among the substituents illustrated above, or being unsubstituted, and R, R' and R" are the same as or different from each other and each independently hydrogen; deuterium; halogen; substituted or unsubstituted alkyl having 1 to 60 carbon atoms; substituted or unsubstituted aryl having 6 to 60 carbon atoms; or substituted or unsubstituted heteroaryl having 2 to 60 carbon atoms.

3. The heterocyclic compound of Claim 1, wherein Chemical Formula 1 is represented by any one of the following Chemical Formulae 1-1 to 1-4: 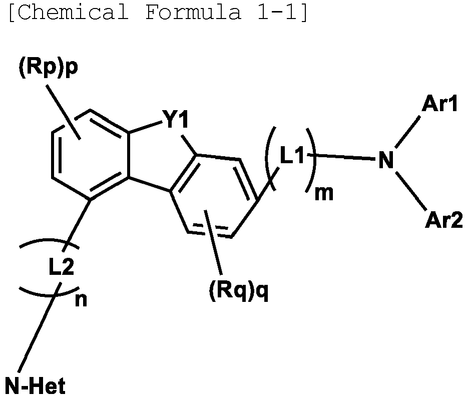 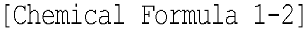 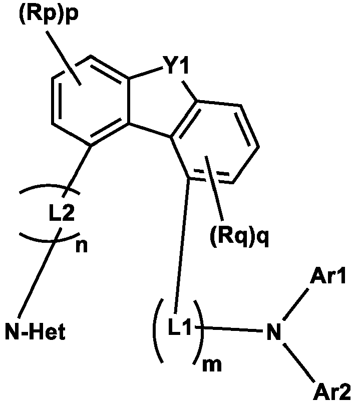 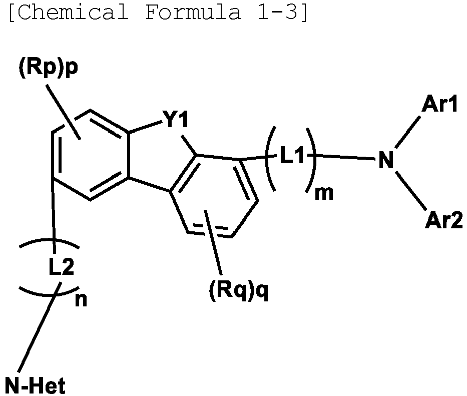 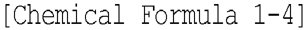 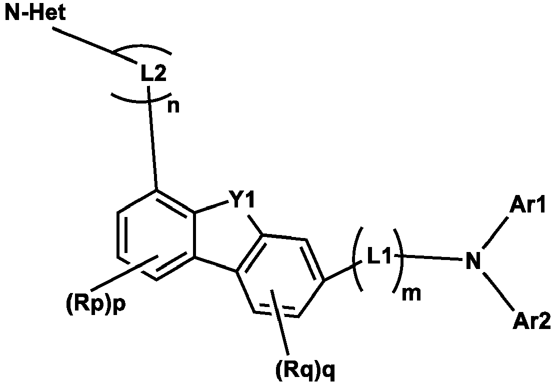
in Chemical Formulae 1-1 to 1-4,
substituents have the same definitions as in Chemical Formula 1.

4. The heterocyclic compound of Claim 1, wherein N-Het is a group represented by the following Chemical Formula 3: 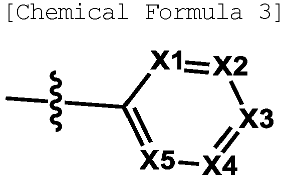
in Chemical Formula 3,
X1 is CR1 or N, X2 is CR2 or N, X3 is CR3 or N, X4 is CR4 or N, X5 is CR5 or N, and at least one of X1 to X5 is N; and
R1 to R5 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; -P(=O)R10R12; and NR13R14, or two or more groups adjacent to each other bond to each other to form a substituted or unsubstituted aliphatic or aromatic hydrocarbon ring or heteroring, R10 to R14 are the same as or different from each other and each independently hydrogen; deuterium; a halogen group; a substituted or unsubstituted alkyl group having 1 to 60 carbon atoms; a substituted or unsubstituted aryl group having 6 to 60 carbon atoms; or a substituted or unsubstituted heteroaryl group having 2 to 60 carbon atoms, and 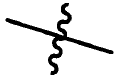 is a site linked to Chemical Formula 1.

5. The heterocyclic compound of Claim 1, wherein Ar1 and Ar2 are the same as or different from each other, and each independently a substituted or unsubstituted phenyl group; a substituted or unsubstituted biphenyl group; a substituted or unsubstituted terphenyl group; a substituted or unsubstituted fluorenyl group; or a substituted or unsubstituted naphthyl group.

6. The heterocyclic compound of Claim 1, wherein Rp and Rq are hydrogen.

7. The heterocyclic compound of Claim 1, wherein Chemical Formula 1 is represented by any one of the following compounds:

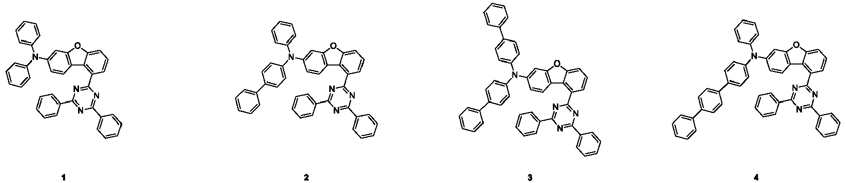

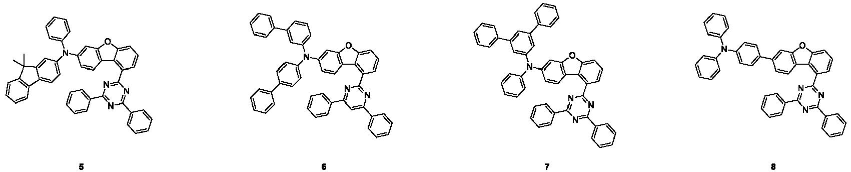

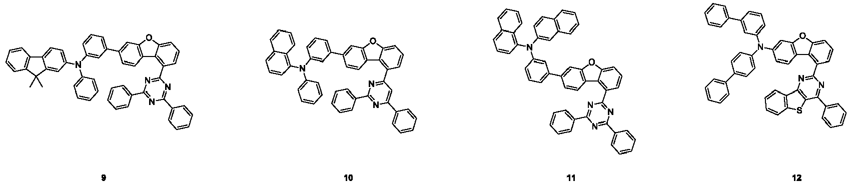

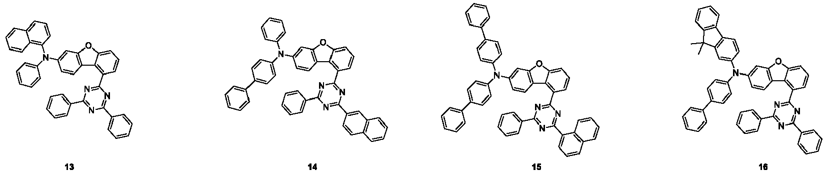

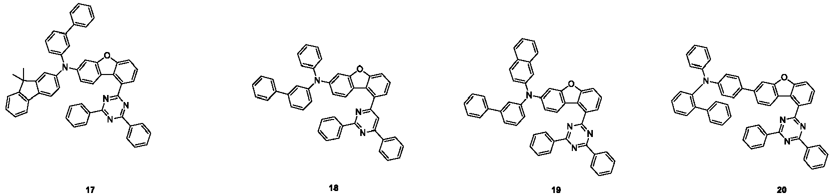

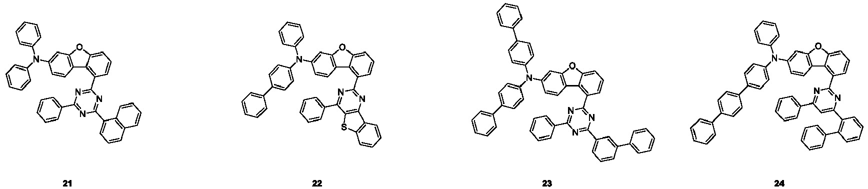

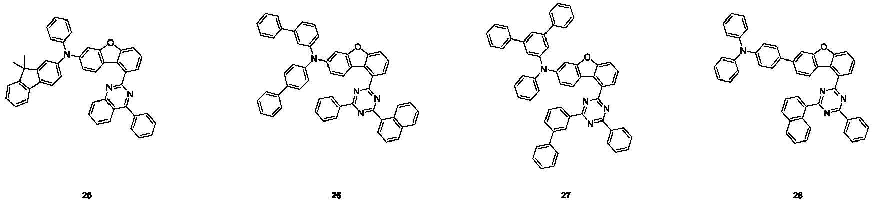

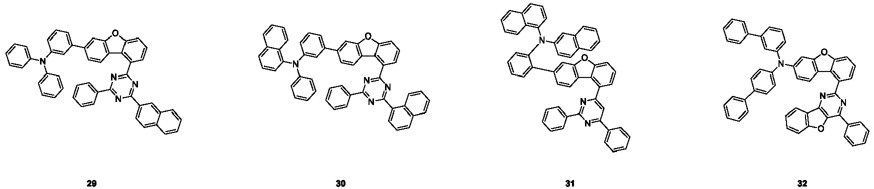

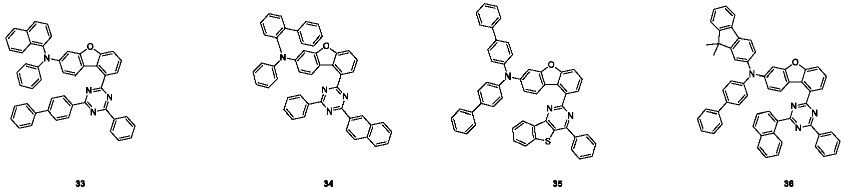

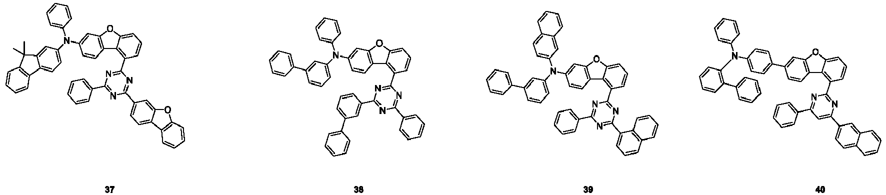

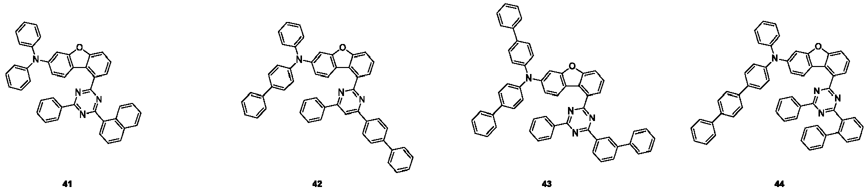

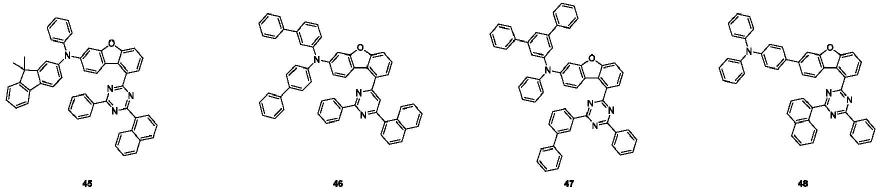

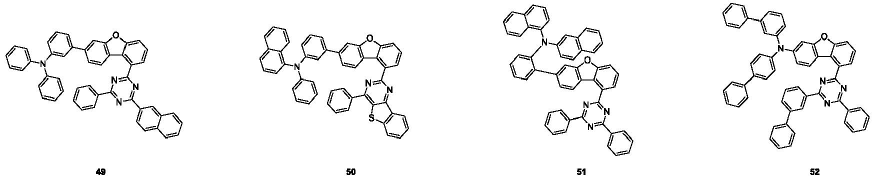

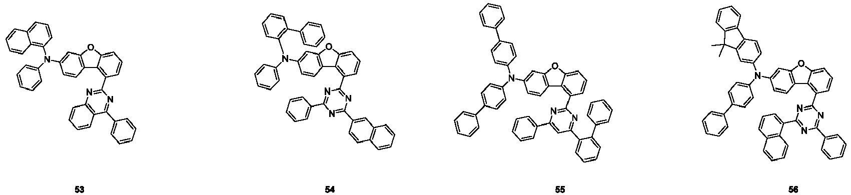

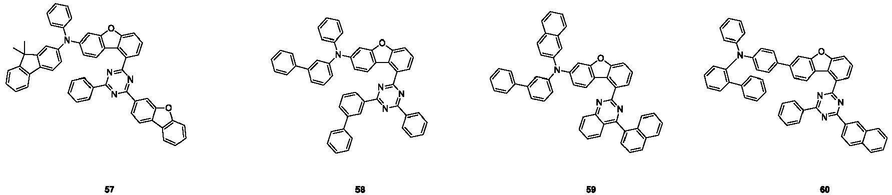

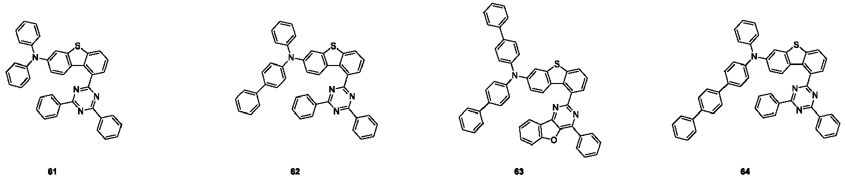

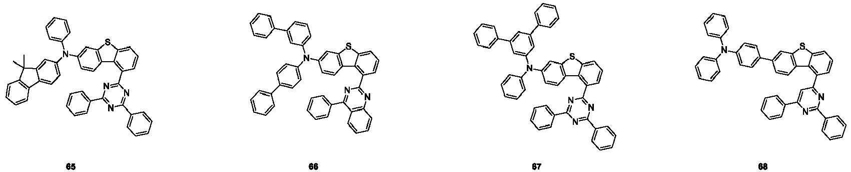

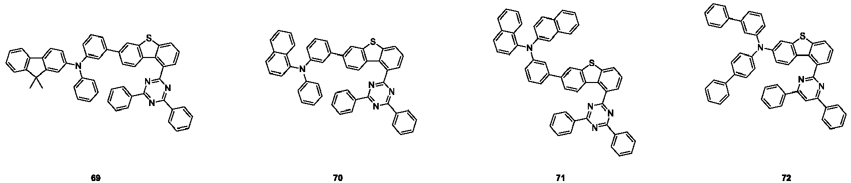

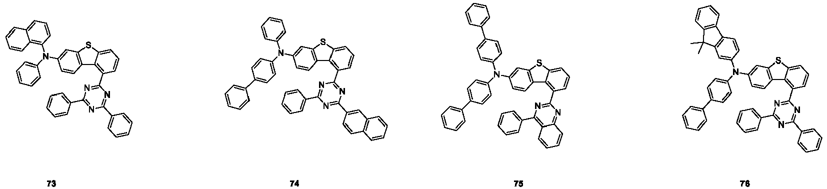

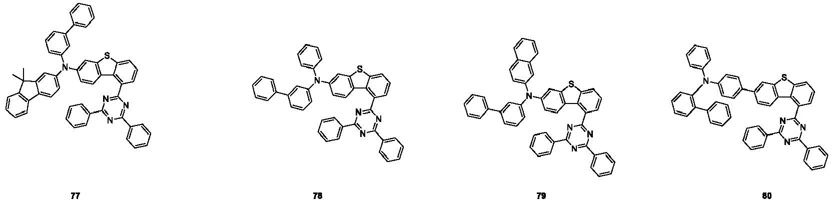

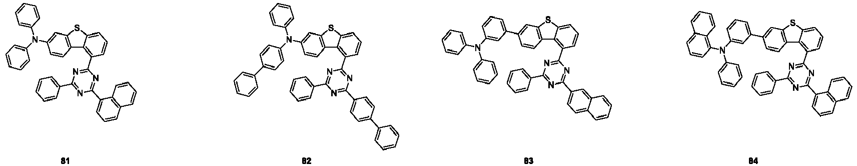

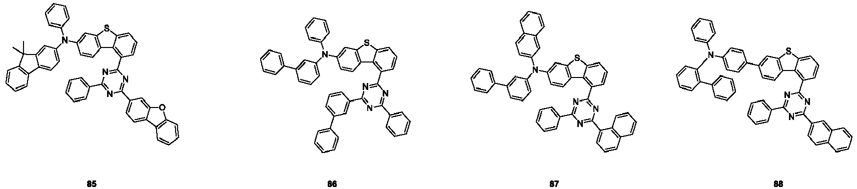

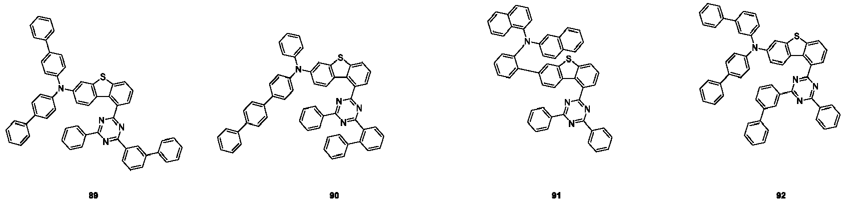

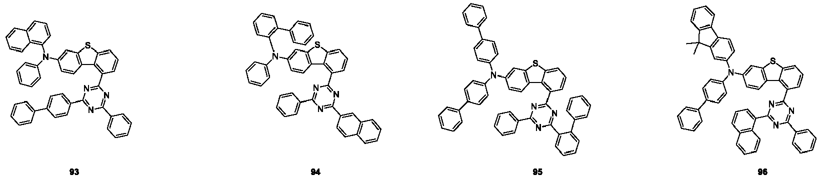

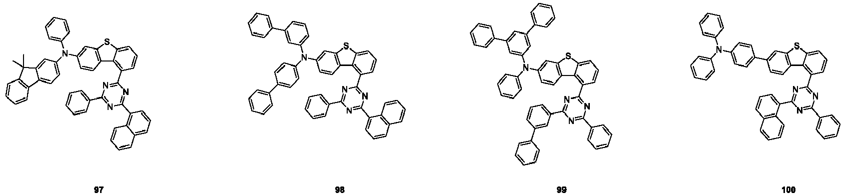

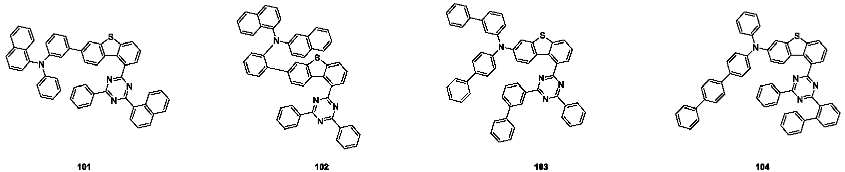

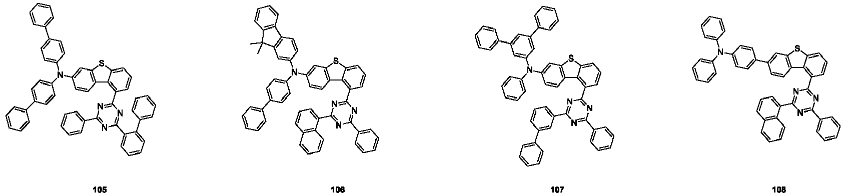

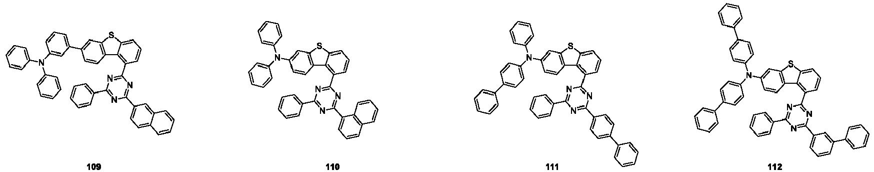

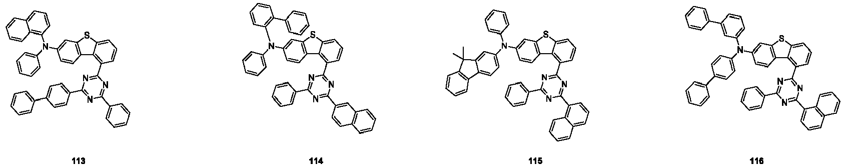

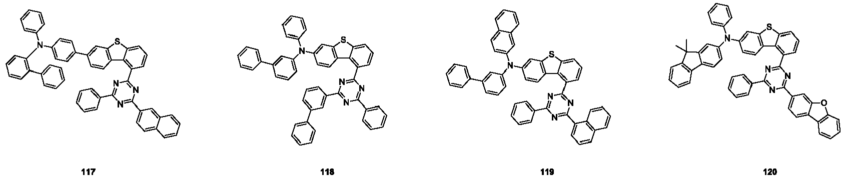

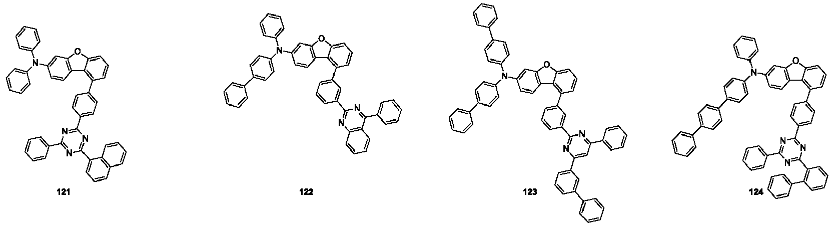

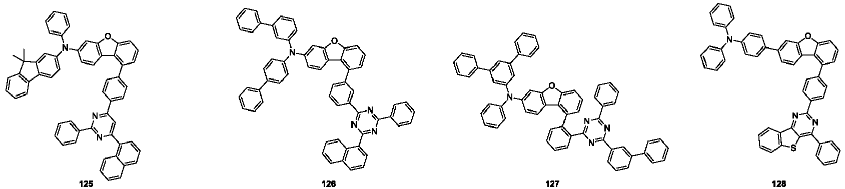

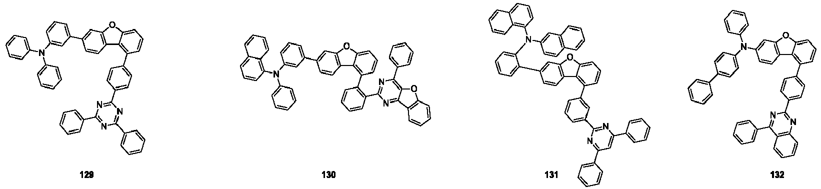

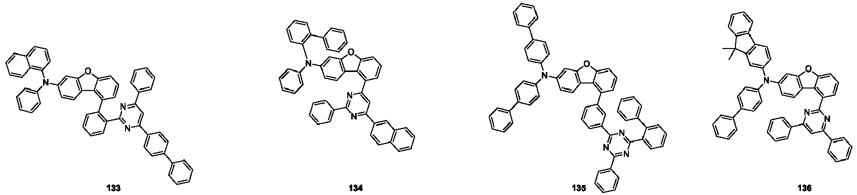

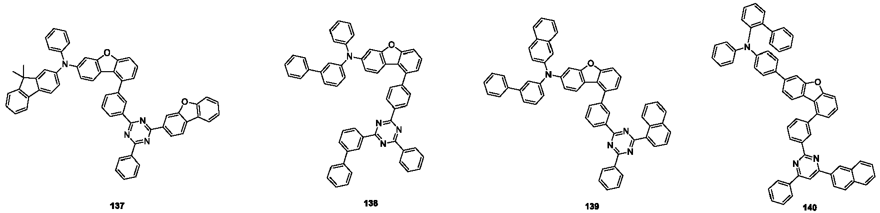

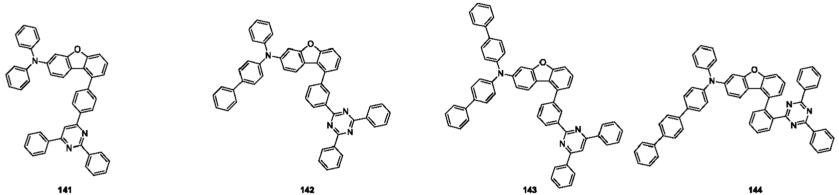

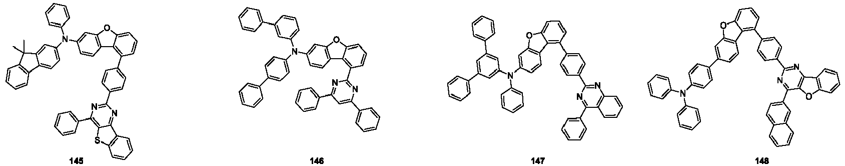

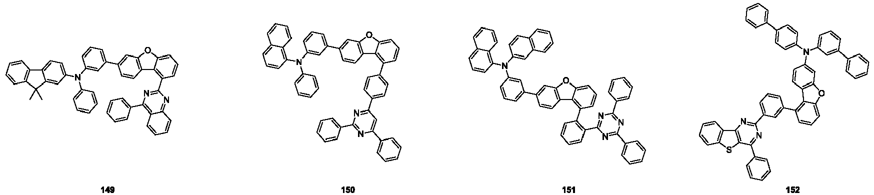

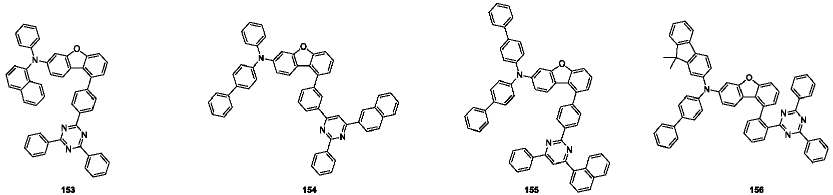

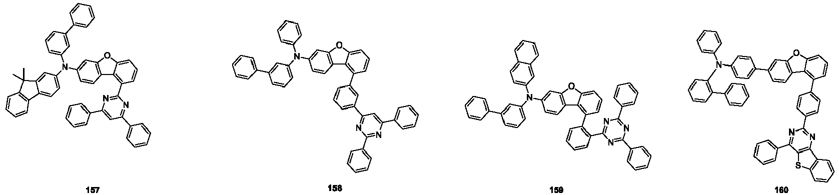

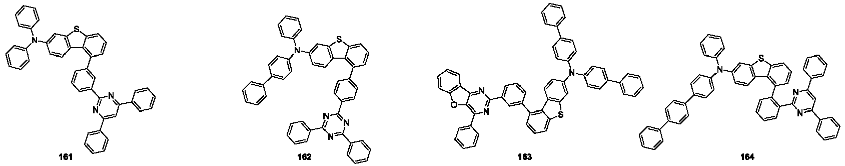

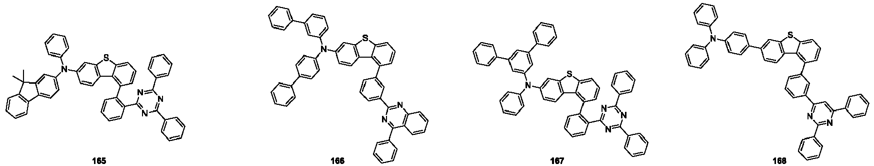

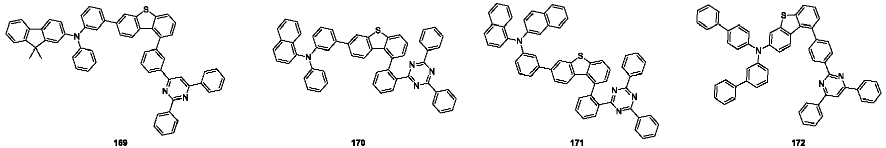

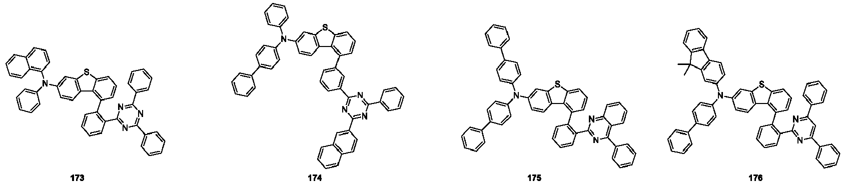

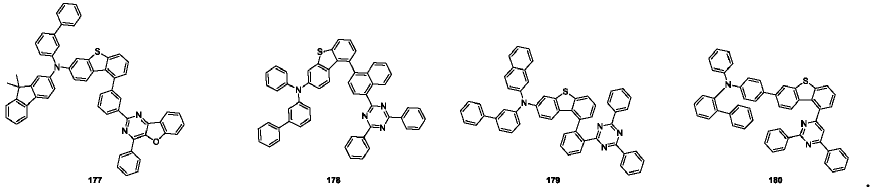

8. An organic light emitting device comprising:
a first electrode;
a second electrode; and
one or more organic material layers provided between the first electrode and the second electrode,
wherein one or more layers of the organic material layers comprise the heterocyclic compound of any one of Claims 1 to 7.

9. The organic light emitting device of Claim 8, wherein the organic material layer comprises one or more light emitting layers, and the light emitting layer comprises the heterocyclic compound.

10. The organic light emitting device of Claim 9, wherein the light emitting layer comprises two or more host materials, and at least one of the host materials comprises the heterocyclic compound as a host material of a light emitting material.

11. The organic light emitting device of Claim 8, further comprising one, two or more layers selected from the group consisting of a light emitting layer, a hole injection layer, a hole transfer layer, an electron injection layer, an electron transfer layer, a hole auxiliary layer and a hole blocking layer.

12. The organic light emitting device of Claim 8, wherein the organic material layer comprises the heterocyclic compound represented by Chemical Formula 1 as a first compound, and further comprises a heterocyclic compound represented by the following Chemical Formula 2 as a second compound: 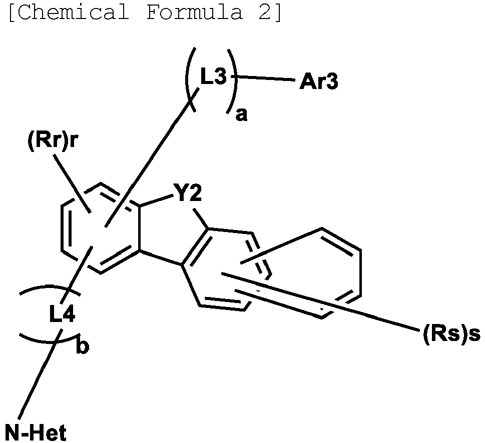
in Chemical Formula 2,
Y2 is O; or S;
L3 and L4 are the same as or different from each other, and each independently a direct bond; a substituted or unsubstituted arylene group having 6 to 60 carbon atoms; or a substituted or unsubstituted heteroarylene group having 2 to 60 carbon atoms, a and b are each an integer of 0 to 3, and when a and b are each 2 or greater, substituents in the parentheses are the same as or different from each other;
N-Het is a monocyclic or polycyclic heterocyclic group substituted or unsubstituted and comprising one or more Ns;
Ar3s are the same as or different from each other, and each independently a substituted or unsubstituted aryl group having 6 to 60 carbon atoms; or a substituted or unsubstituted heteroaryl group having 2 to 60 carbon atoms; and
Rr and Rs are the same as or different from each other, and each independently hydrogen; deuterium; a halogen group; or a substituted or unsubstituted alkyl group having 1 to 60 carbon atoms, r is an integer of 0 to 2, and when r is 2, substituents in the parentheses are the same as or different from each other, s is an integer of 0 to 6, and when s is 2 or greater, substituents in the parentheses are the same as or different from each other.

13. The organic light emitting device of Claim 12, wherein Chemical Formula 2 is represented by any one of the following compounds:

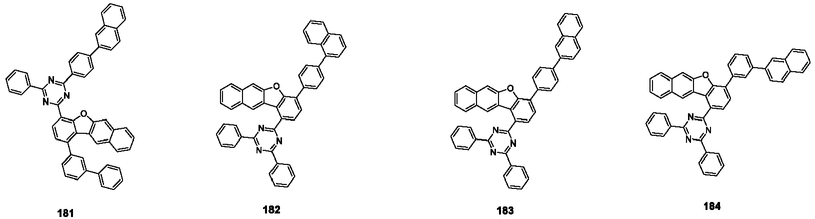

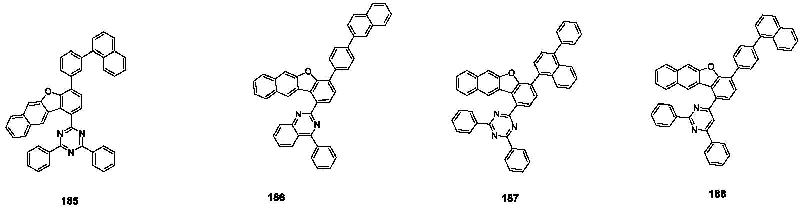

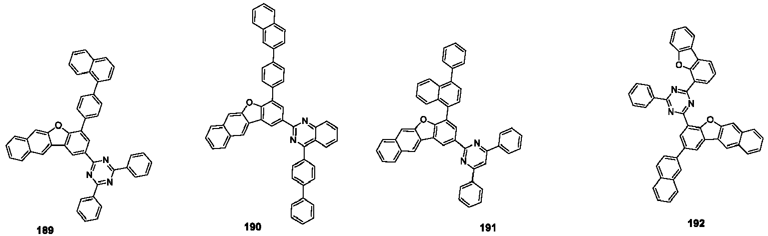

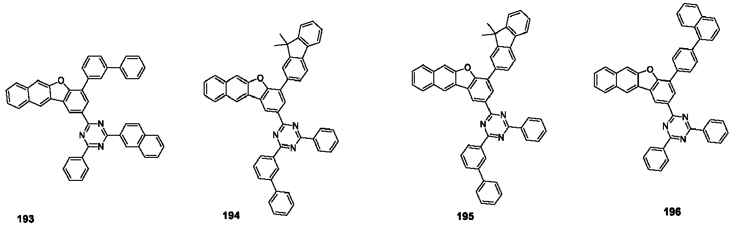

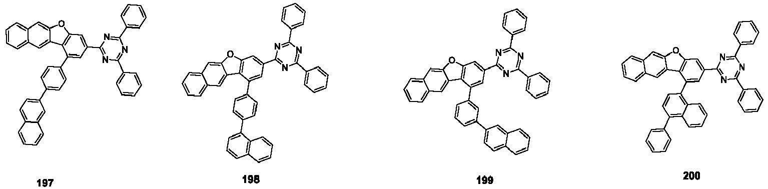

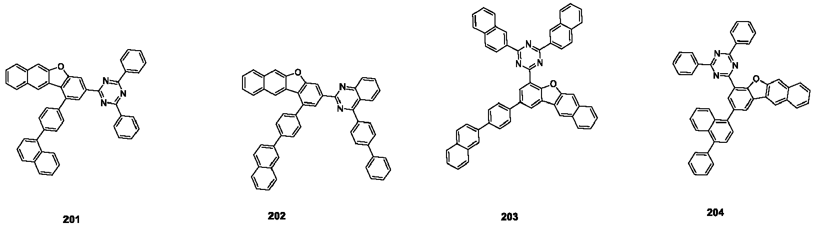

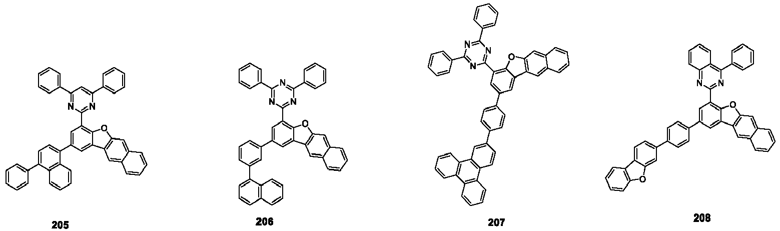

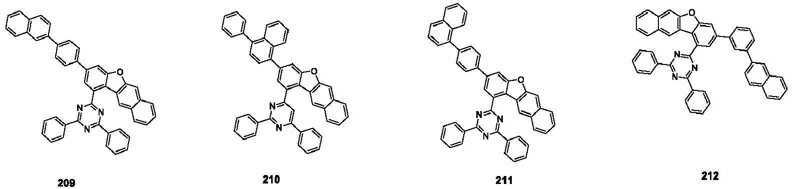

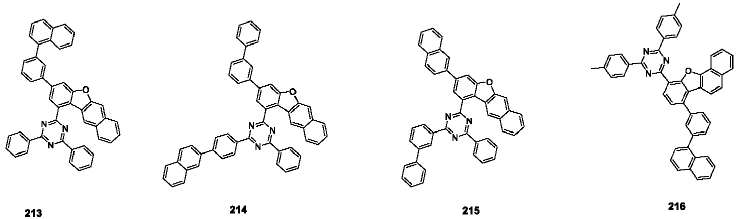

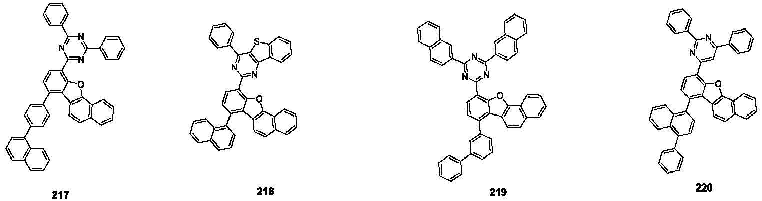

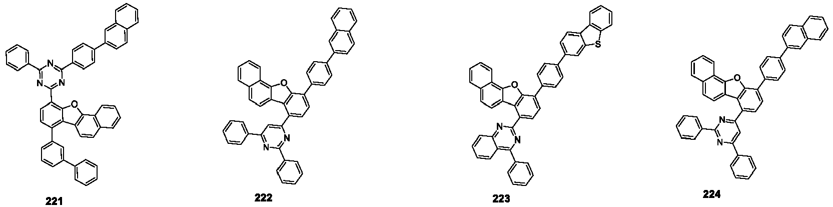

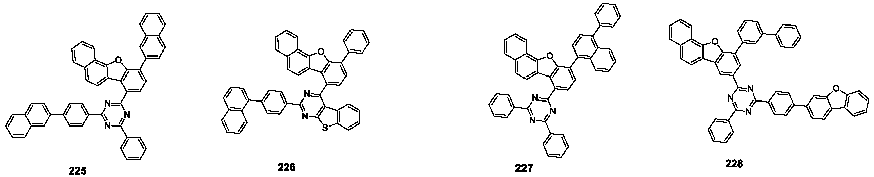

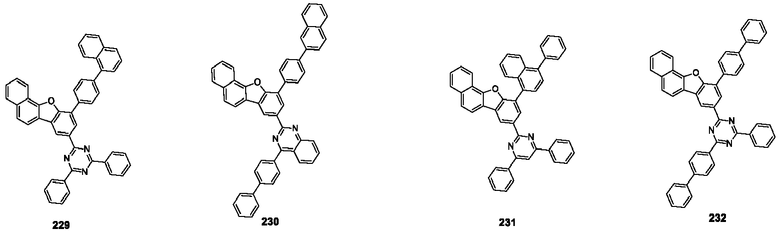

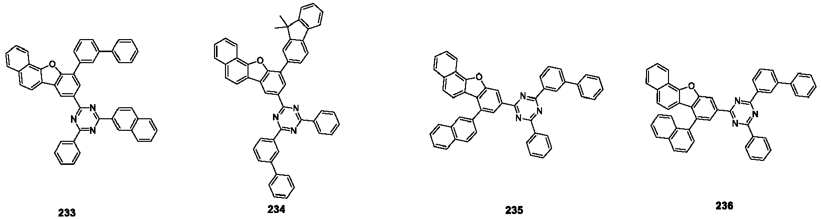

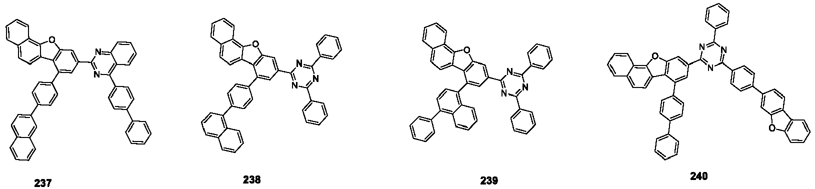

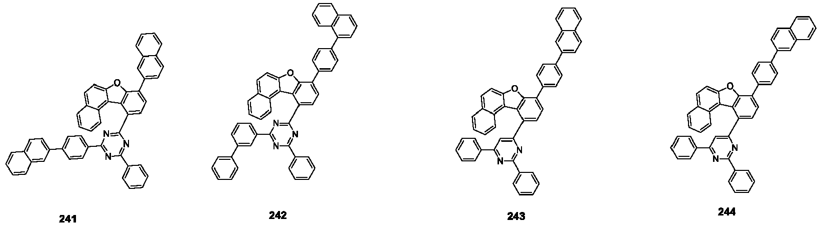

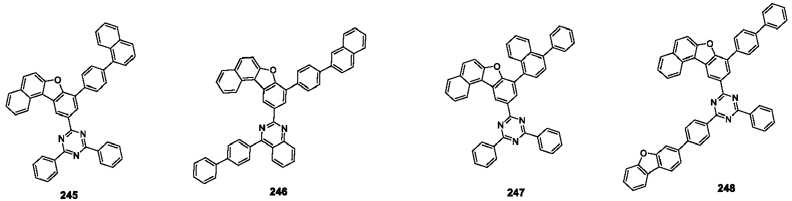

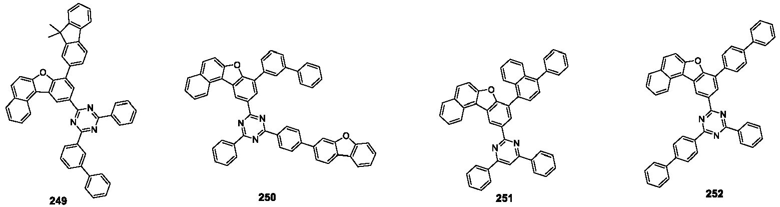

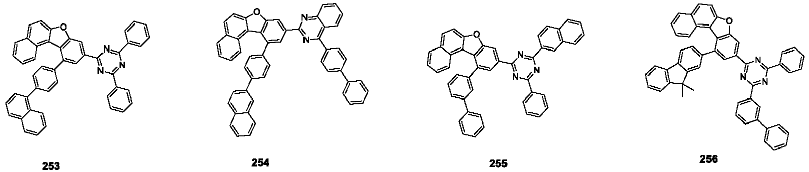

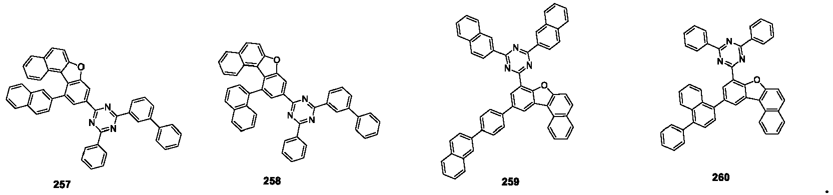

14. A composition for an organic material layer of an organic light emitting device, the composition comprising:
the heterocyclic compound represented by Chemical Formula 1 of Claim 1; and
a heterocyclic compound represented by the following Chemical Formula 2: 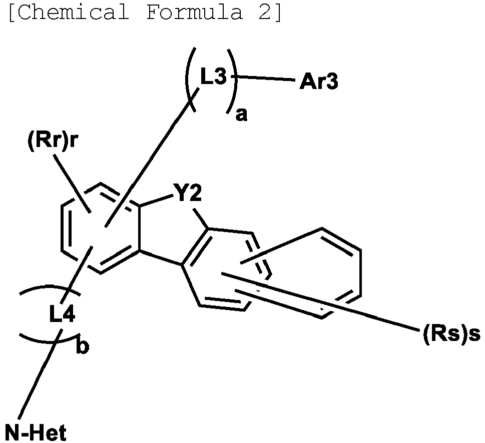
wherein, in Chemical Formula 2,
Y2 is O; or S;
L3 and L4 are the same as or different from each other, and each independently a direct bond; a substituted or unsubstituted arylene group having 6 to 60 carbon atoms; or a substituted or unsubstituted heteroarylene group having 2 to 60 carbon atoms, a and b are each an integer of 0 to 3, and when a and b are each 2 or greater, substituents in the parentheses are the same as or different from each other;
N-Het is a monocyclic or polycyclic heterocyclic group substituted or unsubstituted and comprising one or more Ns;
Ar3s are the same as or different from each other, and each independently a substituted or unsubstituted aryl group having 6 to 60 carbon atoms; or a substituted or unsubstituted heteroaryl group having 2 to 60 carbon atoms; and
Rr and Rs are the same as or different from each other, and each independently hydrogen; deuterium; a halogen group; or a substituted or unsubstituted alkyl group having 1 to 60 carbon atoms, r is an integer of 0 to 2, and when r is 2, substituents in the parentheses are the same as or different from each other, s is an integer of 0 to 6, and when s is 2 or greater, substituents in the parentheses are the same as or different from each other.

15. The composition for an organic material layer of an organic light emitting device of Claim 14, wherein, in the composition, the heterocyclic compound represented by Chemical Formula 1:the heterocyclic compound represented by Chemical Formula 2 have a weight ratio of 1:10 to 10:1.
